(19) Europäisches Patentamt
European Patent Office
Office européen des brevets

(11) EP 4 410 826 A1

(12) EUROPEAN PATENT APPLICATION
published in accordance with Art. 153(4) EPC

(43) Date of publication:
**07.08.2024 Bulletin 2024/32**

(21) Application number: **22875182.2**

(22) Date of filing: **30.09.2022**

(51) International Patent Classification (IPC):
***C07K 14/54*** (2006.01) ***C07K 1/02*** (2006.01)
***A61P 35/00*** (2006.01) ***A61P 31/00*** (2006.01)
***A61K 38/20*** (2006.01)

(52) Cooperative Patent Classification (CPC):
**A61K 9/00; A61K 9/08; A61K 9/19; A61K 38/20; A61K 47/60; A61P 7/00; A61P 11/00; A61P 31/00; A61P 31/04; A61P 31/06; A61P 31/12; A61P 31/14; A61P 31/18; A61P 31/20; A61P 31/22;** (Cont.)

(86) International application number:
**PCT/CN2022/123400**

(87) International publication number:
**WO 2023/051801 (06.04.2023 Gazette 2023/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2021 CN 202111166180**

(71) Applicant: **Kanglitai Biomedical (Qingdao) Co., Ltd.**
**Qingdao, Shandong 266111 (CN)**

(72) Inventors:
- **QIAO, Tiankui**
  **Qingdao, Shandong 266111 (CN)**
- **ZHENG, Binbin**
  **Qingdao, Shandong 266111 (CN)**
- **CHEN, Wencheng**
  **Qingdao, Shandong 266111 (CN)**
- **YAN, Lele**
  **Qingdao, Shandong 266111 (CN)**
- **WANG, Jiangang**
  **Qingdao, Shandong 266111 (CN)**
- **WU, Mingyuan**
  **Qingdao, Shandong 266111 (CN)**

(74) Representative: **Herrero & Asociados, S.L.**
**Edificio Aqua - Agustín de Foxá, 4-10**
**28036 Madrid (ES)**

# (54) POLYETHYLENE GLYCOL DERIVATIVE MODIFIED INTERLEUKIN-12, PREPARATION METHOD THEREFOR AND APPLICATION THEREOF

(57) Polyethylene glycol derivative modified interleukin-12 (IL-12), a preparation method thereof and an application thereof. The polyethylene glycol derivative modified IL-12 is obtained by performing a modification reaction on a polyethylene glycol derivative and IL-12. Active groups of the polyethylene glycol derivative comprise one or more among an aldehyde group, ketone group, maleimide group, vinyl sulfone group, terminal alkyne group, succinimide group, p-nitrophenyl group, isobutyl chloroformate group, halo group, thio group and carboxyl group. Active groups of the IL-12 comprise one or more among an N-terminal α-amino group, ε-amino group of a lysine side chain and free sulfhydryl group of cysteine. IL-12 that has passed through polyethylene glycol derivative modification may balance the drug activity and drug toxicity of IL-12, and a larger dose may be used in a single dose. In addition, the IL-12 may maintain an effective blood concentration in the body for a longer period of time without causing serous toxic side effects, which is beneficial for the continuity of treatment and improves the treatment effects of diseases.

EP 4 410 826 A1

(52) Cooperative Patent Classification (CPC): (Cont.)
**A61P 33/00; A61P 33/02; A61P 33/06;**
**A61P 33/12; A61P 35/00; A61P 37/04;**
**C07K 1/02; C07K 1/107; C07K 14/54**

## Description

### Technical Field

The present invention belongs to the technical field of biopharmaceuticals and relates to a polyethylene glycol derivative modified interleukin-12, a preparation method thereof and an application thereof.

### Background Art

Interleukin-12 (IL-12), as a cytokine with broad biological activities, plays an indispensable role in immune regulation. IL-12 can promote the differentiation of Th0 cells into Th1 cells, prevent the development of Th0 cells into Th2 cells, induce T cells and NK cells to produce several cytokines: IFN-$\gamma$, IFN-$\alpha$, GM-CSF, M-CSF, IL-13, IL-8, and IL-2, act through these mediators to inhibit the secretion of IL-4 by Th2 cells, *etc.* IL-12 can also induce enhanced cellular toxicity, which is weaker than that induced by IL-2 but comparable to that induced by IFN-y. When IL-12 works in conjunction with IL-2, the most significant enhancement of NK cell-mediated cytotoxicity can be observed, which is an additive effect rather than a synergistic effect between the two factors. IL-12 plays an important regulatory role in both innate immunity and adaptive immunity. Therefore, IL-12 has obvious application value in the body's resistance to infection by various bacteria, viruses, fungi, and parasites, as well as in anti-tumor aspect. In addition, IL-12 also has a regulatory effect on hematopoietic cells. IL-12 works synergistically with iron factor (SF), IL-13 and other hematopoietic cell growth factors to increase the survival rate and proliferation of hematopoietic stem cells in the broth, and to increase the number and size of colonies of early pluripotent hematopoietic stem cells (including cells with bipotential for bone marrow B-lymphoid systems). Therefore, IL-12 also has great positive significance in the hemogram recovery and can be used to improve the side effects of abnormal hemogram caused by radiotherapy and chemotherapy, as well as for the prevention and treatment of acute radiation syndrome caused by nuclear leakage accidents and nuclear warfare.

Interleukin-12 itself, as a protein, also has many defects in its application as a drug. The half-life of interleukin-12 in the body is short, only 5.3 to 9.6 hours. In addition, systemic administration of interleukin-12 would also cause many side effects, such as: fever/chills, fatigue, nausea, vomiting, headache, especially when the dose of IL-12 is increased, the side effects increase significantly. In previous clinical trials, continuous administration of 500 ng/Kg/day of IL-12 to patients would cause serious toxic side effects (Effects of Single-Dose Interleukin-12 Exposure on Interleukin-12-Associated Toxicity and Interferon-g Production). The toxic side effects of IL-12 are primarily related to the excess production of interferon gamma by T cells or NK cells stimulated by IL-12. In order to cope with the increase in toxic side effects, the dose of IL-12 in general treatment is low. However, the use of IL-12 at lower doses requires frequent injections to maintain blood concentration, due to IL-12's short half-life. Although frequent injections of IL-12 can maintain the blood concentration, it poses significant inconvenience to the patient. Moreover, the frequently violent fluctuations in IL-12 levels in the blood are not conducive to treatment. Too long an interval between injections results in discontinuous treatment and poorer treatment effectiveness, while too short an interval between injections can lead to cumulative time toxicity.

Polyethylene glycol (PEG), also known as polyoxyethylene alcohol or polyoxyethylene glycol, is synthesized by the polymerization of ethylene oxide with ethylene glycol (or diethylene glycol) under the catalysis of a basic catalyst. Due to its stable chemical properties and good biological safety, it has been used in the medical field for a long time. Polyethylene glycol-modified proteins or polypeptide drugs prolong the drug's blood half-life *in vivo,* stabilize the drug, and suppress immunogenicity in various ways.

CN 1186121 A discloses a construction of a eukaryotic expression vector, with the IL-12 gene carried by PEG. This method involves using PEG to carry the IL-12 gene and transfecting it into eukaryotic cells to translate and express the synthetic IL-12 protein, and secreting the protein out of the cells to exert its efficacy. However, the physical carrying is not as strong and stable as the chemical bond and needs to go through more steps, the drug carried is slow to take effect, and the final expression level of IL-12 is also difficult to control. If the expression level is too low, the drug effect would unclear. If the expression level is too high, drug side effects increase. In addition, IL-12 itself is a non-monodisperse macromolecule containing multiple amino acid chains and has a short metabolic half-life in the body. Even if IL-12 is expressed, IL-12 protein alone also has the defects of small therapeutic window and great toxic side effects in therapy as a drug.

CN 109438568 A discloses a method for preparing monodisperse polyethylene glycol monomethyl ether-modified interleukin IL-12 prodrug. Specifically, in this method, interleukin-12, an alkali, and a solvent are added to monodisperse polyethylene glycol monomethyl ether lysine active ester, and the mixture is reacted at 50°C to obtain monodisperse polyethylene glycol monomethyl ether lysine interleukin-12 prodrug; In this method, although the molecular weight of the product is increased by connecting interleukin-12 with polyethylene glycol through chemical bonds, the molecular weight of the polyethylene glycol used is small. In order to achieve a good effect of prolonging the half-life, this method uses polyethylene glycol to modify IL-12 randomly at multiple sites. Random modifications at multiple sites

tend to mask more surface of IL-12 and reduce the activity of IL-12 further. Moreover, the reaction is carried out in an organic solvent in an alkaline environment at 50°C, which is extremely unfriendly to the protein IL-12, easily cause protein denaturation and inactivation, and leads to further decline in IL-12 activity. Further, random modification leads to complex products, posing greater potential risks when used as drugs.

Summary of the Invention

Based on the defects existing in the prior art, the first purpose of the present invention is to provide a polyethylene glycol derivative modified interleukin-12. By improving the structure of interleukin-12, its side effects in clinical application are reduced. The second purpose of the present invention is to provide a method for preparing the polyethylene glycol derivative modified interleukin-12. By controlling the reaction conditions and preparation conditions, an interleukin-12 site-specifically modified at single site with polyethylene glycol derivative or an interleukin-12 reversibly modified at multiple sites can be obtained. The third purpose of the present invention is to provide the application of the polyethylene glycol derivative modified interleukin-12 in the preparation of an immunity-improving drug and an anti-infective drug; The fourth purpose of the present invention is to provide the application of the polyethylene glycol derivative modified interleukin-12 in the preparation of a medicament for preventing or treating an disease with abnormal hemogram; The fifth purpose of the present invention is to provide the application of the polyethylene glycol derivative modified interleukin-12 in the preparation of an anti-tumor drug for preventing or treating a tumor; The sixth purpose of the present invention is to provide a pharmaceutical preparation containing the polyethylene glycol derivative modified interleukin-12.

In an aspect, the present invention provides a polyethylene glycol derivative modified interleukin-12, which is obtained by performing a modification reaction on a polyethylene glycol derivative and interleukin-12; wherein, an active group of the polyethylene glycol derivative and an active group of the interleukin-12 participate in the modification reaction. The active group of the polyethylene glycol derivative comprises one or more among a aldehyde group, a ketone group, a maleimide group, a vinyl sulfone group, a terminal alkyne group, a succinimide group, a p-nitrophenyl group, an isobutyl chloroformate group, a halo group (haloalkyl group), a thio group and a carboxyl group, but not limited to these. The active group of the interleukin-12 comprises one or more among an N-terminal $\alpha$-amino group, an $\varepsilon$-amino group of a lysine side chain and a free sulfhydryl group of a cysteine, but not limited to these.

In the present invention, the polyethylene glycol derivative and IL-12 are coupled through covalent bonds to obtain the interleukin-12 with polyethylene glycol derivative modification introduced, so that the interleukin-12 has more advantages of becoming a potential drug product while maintaining the original biological activity of the interleukin-12. The advantages comprise, but are not limited to, increased molecular weight and hydration radius, improved *in vivo* stability, prolonged *in vivo* half-life, reduced immunogenicity, improved storage stability, storage capacity, and safe use as a liquid preparation, a frozen formulation, and a dried lyophilized formulation after several days. Polyethylene glycol derivative modified interleukin-12 can not only prolong the half-life, but also balance the drug activity and drug toxicity of IL-12. The toxic side effects of IL-12 are mainly caused by excessive interferon $\gamma$ produced by T cells or NK cells stimulated by IL-12. A little excessive IL-12 would bring great toxic side effects, which is very troublesome for treatment, and the dose is difficult to control. When natural IL-12 is used for treatment, the toxic side effects can only be reduced by reducing the dose of a single injection. However, the lower dose makes it difficult to maintain the therapeutic effect of the drug, and frequent low-dose injections are needed to maintain blood concentration. Polyethylene glycol derivative modified interleukin-12 can reduce the toxic side effects of IL-12 and balance the drug activity and drug toxicity, and a larger dose may be used in a single dose. In addition, the IL-12 may maintain an effective blood concentration in the body for a longer period of time without causing serous toxic side effects, which is beneficial for the continuity of treatment and improves the treatment effects of diseases.

Interleukin-12 in the present invention may further comprise mutants, monomers, polymers, bioactive peptides, functional fragments, functional derivatives and fusion proteins of interleukin-12, subunits P40 and P35 that constitute interleukin-12, mutants, monomers, polymers, bioactive peptides, functional fragments, functional derivatives and fusion proteins of subunits P40 and P35 of interleukin-12, *etc.*

In the above-mentioned polyethylene glycol derivative modified interleukin-12, preferably, the active group of the polyethylene glycol derivative is an aldehyde group, a ketone group, a succinimide group, an isobutyl chloroformate group, a p-nitrophenyl group or a carboxyl group, the active group of the interleukin-12 that undergoes the modification reaction is an N-terminal $\alpha$-amino group (any one or both of the N-terminal $\alpha$-amino groups); or

the active group of the polyethylene glycol derivative is a maleimide group, a vinyl sulfone group, a terminal alkyne group, a halo group or a thio group, and the active group of the interleukin-12 that undergoes the modification reaction is a free sulfhydryl group of a cysteine; preferably a free sulfhydryl group of a cysteine at the position C252 of the P40 subunit; or

The active group of the polyethylene glycol derivative is a succinimide group, an isobutyl chloroformate group, a p-nitrophenyl group or a carboxyl group, and the active group of the interleukin-12 that undergoes the modification reaction is an $\varepsilon$-amino group of a lysine side chain (any one or more of the $\varepsilon$-amino groups of multiple lysine side chains).

In the above-mentioned polyethylene glycol derivative modified interleukin-12, preferably, the molecular weight of the polyethylene glycol derivative is 5-40 KD; preferably 10-30 KD; and more preferably, 20 KD.

Among the above-mentioned polyethylene glycol derivative modified interleukin-12, preferably, the polyethylene glycol derivative comprises one or more of a polyethylene glycol aldehyde derivative, a polyethylene glycol ketone derivative, a polyethylene glycol active ester derivative, a polyethylene glycol carboxyl derivative, a polyethylene glycol unsaturated bond-containing derivative, a polyethylene glycol haloalkane derivative, a polyethylene glycol sulfur activity-containing derivative; but not limited to these. Preferably, the polyethylene glycol aldehyde derivative comprises one or more of a polyethylene glycol acetaldehyde derivative, a polyethylene glycol propionaldehyde derivative and a polyethylene glycol butyraldehyde derivative; but not limited to these. The polyethylene glycol propionaldehyde derivative comprises monomethoxy polyethylene glycol propionaldehyde and/or two-arm polyethylene glycol propionaldehyde; but not limited to these. The polyethylene glycol active ester derivative comprises one or more of a polyethylene glycol succinimide active ester derivative, a polyethylene glycol p-nitrobenzene active ester derivative and a polyethylene glycol isobutyl chloroformate active ester derivative; but not limited to these. The polyethylene glycol succinimide active ester derivative comprises monomethoxy polyethylene glycol succinimide succinate and/or monomethoxy polyethylene glycol succinimide carbonate; but not limited to these. The polyethylene glycol carboxyl derivative comprises one or more of a polyethylene glycol acetic acid derivative, a polyethylene glycol propionic acid derivative, a polyethylene glycol butyric acid derivative, a polyethylene glycol valeric acid derivative, a polyethylene glycol hexanoic acid derivative, a polyethylene glycol oxalic acid derivative, a polyethylene glycol malonic acid derivative, a polyethylene glycol succinic acid derivative, a polyethylene glycol glutaric acid derivative and a polyethylene glycol hexanedioic acid derivative; but not limited to these. The polyethylene glycol carboxyl derivative comprises monomethoxy polyethylene glycol acetic acid. The polyethylene glycol unsaturated bond-containing derivative comprises one or more of polyethylene glycol vinyl sulfones, polyethylene glycol maleimides, and polyethylene glycol terminal acetylenes; but not limited to these. the polyethylene glycol unsaturated bond-containing derivative comprises one or more of a monomethoxy polyethylene glycol vinyl sulfone, a monomethoxy polyethylene glycol maleimide and a two-arm polyethylene glycol maleimide; but not limited to these. the polyethylene glycol haloalkane derivative comprises one or more of a polyethylene glycol iodopropionamide derivative, a polyethylene glycol iodoacetamide derivative, a polyethylene glycol chloropropionamide derivative, a polyethylene glycol chloroacetamide derivative, a polyethylene glycol bromopropionamide derivative, a polyethylene glycol bromoacetamide derivative, a polyethylene glycol fluoropropionamide derivative and a polyethylene glycol fluoroacetamide derivative; but not limited to these. The polyethylene glycol haloalkane derivative comprises monomethoxy polyethylene glycol iodoacetamide. The polyethylene glycol sulfur activity-containing derivative comprises one or more of polyethylene glycol dithioisopyridines, polyethylene glycol thiosulfonates, and polyethylene glycol thiols; but not limited to these. The polyethylene glycol sulfur activity-containing derivative comprises one or more of monomethoxy polyethylene glycol dithioisopyridine, monomethoxy polyethylene glycol thiomethanesulfonate and monomethoxy polyethylene glycol thiobenzenesulfonate; but not limited to these.

According to a specific embodiment of the present invention, the polyethylene glycol derivative modified interleukin-12 of the present invention comprises one or more of the following substances: monomethoxy polyethylene glycol propionaldehyde-modified interleukin-12, two-arm polyethylene glycol propionaldehyde-modified interleukin-12, monomethoxy polyethylene glycol succinimide succinate-modified interleukin-12, monomethoxy polyethylene glycol maleimide-modified interleukin-12, branched monomethoxy polyethylene glycol maleimide-modified interleukin-12, monomethoxy polyethylene glycol succinimide carbonate-modified interleukin-12, monomethoxy polyethylene glycol succinimide carbonate-modified interleukin-12, monomethoxy polyethylene glycol vinyl sulfone-modified interleukin-12, monomethoxy polyethylene glycol iodoacetamide-modified interleukin-12, monomethoxy polyethylene glycol dithioisopyridine-modified interleukin-12, monomethoxy polyethylene glycol acetic acid-modified interleukin-12.

In another aspect, the present invention further provides a method for preparing the above-mentioned polyethylene glycol derivative modified interleukin-12, and the method comprises:

dissolving interleukin-12 in a solvent to prepare an interleukin-12 solution;
dissolving a polyethylene glycol derivative in water to prepare an aqueous solution of the polyethylene glycol derivative;
adding the aqueous solution of the polyethylene glycol derivative into the interleukin-12 solution dropwise for a reaction under stirring;
performing ultrafiltration to remove impurities after the reaction, and then performing separation and purification by chromatography to obtain the polyethylene glycol derivative modified interleukin-12.

In the preparation method of the present invention, an IL-12 site-specifically modified at single site with polyethylene glycol derivative or an IL-12 reversibly modified at multiple sites can be obtained by controlling the reaction conditions. Modification at single site occupies less surface area of the protein than modification at multiple sites, offering a more distinct modification site. At the same time, a single polyethylene glycol derivative with a larger molecular weight

is used, the biological activity of IL-12 can be better retained when the molecular weight of the product increases significantly and appropriately reduced activity also makes it easier to combine with other drugs. The IL-12 reversibly modified at multiple sites has a larger molecular weight and longer blood drug half-life when used. PEG can be dissociated again to restore the activity of IL-12. At the same time, the reaction of the present invention is carried out at low temperature or normal temperature, which can better retain the biological activity of IL-12. This step is performed in particular as follows: when a polyethylene glycol aldehyde derivative is used as the polyethylene glycol derivative, IL-12 only has two subunits, but it also has 39 lysine side chain amino groups, and by controlling the reaction conditions, it is able to achieve selective site-specific coupling of the polyethylene glycol aldehyde derivative to the terminal $\alpha$-amino group of interleukin-12, rather than random modification of the terminal $\alpha$-amino group or the $\varepsilon$-amino group of the lysine side chains of interleukin-12. when a polyethylene glycol active ester derivative and a polyethylene glycol carboxyl derivative are used as polyethylene glycol derivatives, by controlling the reaction conditions, it is able to achieve selective site-specific coupling of the polyethylene glycol active ester derivative or the polyethylene glycol carboxyl derivative to the terminal $\alpha$-amino group or the $\varepsilon$-amino group of the lysine side chains of interleukin-12; this type of ester bond-containing linker group can allow the modified interleukin-12 to have the characteristic of reversible modification, and during use, it enables polyethylene glycol to separate from IL-12 again through a hydrolysis reaction, thereby allowing IL-12 to retain more complete biological activity. when a polyethylene glycol unsaturated bond-containing derivative, a polyethylene glycol haloalkane derivative, or a polyethylene glycol sulfur activity-containing derivative is used as the polyethylene glycol derivative, it is able to achieve selective site-specific coupling of the polyethylene glycol unsaturated bond-containing derivative, the polyethylene glycol haloalkane derivative, or the polyethylene glycol sulfur activity-containing derivative to the free sulfhydryl group of cysteine at the position C252 of the P40 subunit of interleukin-12 by controlling the reaction conditions.

Preferably, the chromatography comprises ion exchange chromatography, size exclusion chromatography, hydrophobic chromatography or reversed phase chromatography; but not limited to these.

Preferably, the molar ratio of the polyethylene glycol derivative to the interleukin-12 is (5.5-20) : 1.

Preferably, the polyethylene glycol derivative is a polyethylene glycol aldehyde derivative or a polyethylene glycol ketone derivative, and the solvent comprises an acidic buffer solution with a pH value of 4 to 6.5, preferably an acidic buffer solution with a pH value of 5, so as to control the reaction conditions to be an acidic environment; the temperature of the reaction under stirring is 0-40°C, preferably 10°C; a reducing agent further needs to be added during the reaction.

Preferably, the acidic buffer solution comprises one or more of a MES buffer, a Bis-Tris buffer, a glycine-hydrochloric acid buffer, a disodium hydrogen phosphate-citric acid buffer, a citric acid-sodium citrate buffer, a citric acid-sodium hydroxide-hydrochloric acid buffer, a acetic acid-sodium acetate buffer, a potassium hydrogen phthalate-sodium hydroxide buffer, a disodium hydrogen phosphate-sodium dihydrogen phosphate buffer, a disodium hydrogen phosphate-potassium dihydrogen phosphate buffer and a potassium dihydrogen phosphate-sodium hydroxide buffer, but not limited to these.

Preferably, the reducing agent comprises one or more of sodium cyanoborohydride, sodium borohydride, sodium triacetylborohydride, borane and lutidine borane, but not limited to these; preferably, the reducing agent is sodium cyanoborohydride.

Preferably, the molar ratio of the reducing agent to the interleukin-12 is (30-500) : 1.

Preferably, when the polyethylene glycol derivative is a polyethylene glycol carboxyl derivative, the polyethylene glycol carboxyl derivative needs to be activated with an active condensing agent before reacting with interleukin-12.

Preferably, the active condensing agent comprises one or more of a carbodiimide condensing agent, an onium salt condensing agent and an organophosphorus condensing agent; but not limited to these.

Preferably, the carbodiimide condensing agent comprises one or more of dicyclohexylcarbodiimide, diisopropylcarbodiimide and 1-(3-dimethylaminopropyl)-3-ethylcarbodiimide; but not limited to these.

Preferably, the onium salt condensing agent comprises one or more of 2-(7-azabenzotriazole)-N,N,N',N'-tetramethylurea hexafluorophosphate, benzotriazole-N,N,N',N'-tetramethylurea hexafluorophosphate, 6-chlorobenzotriazole-1,1,3,3-tetramethylurea hexafluorophosphate, O-(N-succinimide)-1,1,3,3-tetramethylurea tetrafluoroborate, 2-(endo-5-norbornene-2,3-dicarboximido)-1,1,3,3-tetramethylurea tetrafluoroborate, O-(7-azabenzotriazol-1-yl)-bis(tetrahydropyrrolyl)carbonium hexafluorophosphate and O-(benzotriazol-1-yl)-N,N,N',N'-dipyrrolylurea hexafluorophosphate; but not limited to these.

Preferably, the organophosphorus condensing agent comprises one or more of diethyl cyanophosphate, diphenyl azidophosphate, diphenyl chlorophosphate, dimethylthiophosphoryl azide, benzotriazole-1-bis(trimethylamino)phosphonium-hexafluorophosphate, 1H-benzotriazole-1-yloxytripyrrolidinyl hexafluorophosphate, triphenylphosphine-polyhalomethane, triphenylphosphine-hexachloroacetone and triphenylphosphine-NBS; but not limited to these.

Preferably, the polyethylene glycol derivative is a polyethylene glycol active ester derivative, and the solvent comprises an alkaline buffer solution with a pH value of 7.5 to 10, preferably an alkaline buffer solution with a pH value of 8, so as to control the reaction conditions to be an alkaline environment; the temperature of the reaction under stirring

is 0-40°C, preferably 10°C.

Preferably, the alkaline buffer solution comprises one or more of a MES buffer, a Bis-Tris buffer, a HEPES buffer, a TEA buffer, a disodium hydrogen phosphate-citric acid buffer, a citric acid-sodium citrate buffer, a citric acid-sodium hydroxide-hydrochloric acid buffer, a acetic acid-sodium acetate buffer, a potassium hydrogen phthalate-sodium hydroxide buffer, a disodium hydrogen phosphate-sodium dihydrogen phosphate buffer, a disodium hydrogen phosphate-potassium dihydrogen phosphate buffer, a potassium dihydrogen phosphate-sodium hydroxide buffer, a sodium barbiturate-hydrochloric acid buffer and a Tris-hydrochloric acid buffer, but not limited these.

Preferably, the polyethylene glycol derivative is a polyethylene glycol carboxyl derivative, a polyethylene glycol unsaturated bond-containing derivative, a polyethylene glycol haloalkane derivative or a polyethylene glycol sulfur activity-containing derivative, and the solvent comprises a neutral or near-neutral buffer solution with a pH value of 6 to 8, preferably a neutral buffer solution with a pH value of 7, so as to control the reaction conditions to be a neutral or near-neutral environment. the temperature of the reaction under stirring is 0-40°C, preferably 10°C.

Preferably, the neutral or near-neutral buffer solution comprises one or more of a MES buffer, a Bis-Tris buffer, a HEPES buffer, a TEA buffer, a disodium hydrogen phosphate-citric acid buffer, a citric acid-sodium citrate buffer, a citric acid-sodium hydroxide-hydrochloric acid buffer, a acetic acid-sodium acetate buffer, a potassium hydrogen phthalate-sodium hydroxide buffer, a disodium hydrogen phosphate-sodium dihydrogen phosphate buffer, a disodium hydrogen phosphate-potassium dihydrogen phosphate buffer, a potassium dihydrogen phosphate-sodium hydroxide buffer, a sodium barbiturate-hydrochloric acid buffer and a Tris-hydrochloric acid buffer; but not limited to these.

In another aspect, the present invention further provides the application of the above-mentioned polyethylene glycol derivative modified interleukin-12 in the preparation of an immunity-improving drug and an anti-infective drug;

Preferably, the resistance to infection comprises resistance to one or more of viral infection diseases, bacterial infection diseases and parasitic infection diseases, but not limited to these; the resistance to viral infection diseases comprise resistance to one or more of HSV, HIV, hepatitis B and hepatitis C, but not limited to these; the resistance to bacterial infection diseases comprise resistance to one or more of pulmonary tuberculosis, salmonellosis and listeriosis, but not limited to these; the resistance to parasitic infection diseases comprise resistance to one or more of malaria, leishmaniasis and schistosomiasis, but not limited to these.

In another aspect, the present invention further provides the application of the above-mentioned polyethylene glycol derivative modified interleukin-12 in the preparation of a medicament with one or more of the following efficacies: improving immunity; resistance to infection ; preventing and/or treating a disease with abnormal hemogram; and preventing and/or treating a tumor. Accordingly, the present invention provides a corresponding method for treating one or more of the following diseases, which method comprises administering an effective amount of the polyethylene glycol derivative modified interleukin-12 of the present invention to a subject in need: improving immunity; resistance to infection ; preventing and/or treating a disease with abnormal hemogram; and preventing and/or treating a tumor.

Preferably, the disease with abnormal hemogram comprises one or more of a disease with abnormal hemogram caused by a hematopoietic malignant tumor, a disease with abnormal hemogram caused by radiotherapy, chemotherapy, or myeloablative therapy, and a disease with abnormal hemogram caused by acute radiation syndrome; but not limited to these.

Preferably, the disease with abnormal hemogram caused by a hematopoietic malignant tumor comprises one or more of chronic myeloid leukemia, chronic lymphocytic leukemia, mantle cell lymphoma, low-grade non-Hodgkin lymphoma, acute myeloid leukemia, moderate lymphoma, multiple myeloma, myelodysplastic syndrome, and Hodgkin's disease; but not limited to these.

Preferably, the tumor comprises various types of solid tumors and/or non-solid tumors.

In another aspect, the present invention further provides a pharmaceutical preparation, which comprises an effective amount of the above-mentioned polyethylene glycol derivative modified interleukin-12. A pharmaceutically acceptable excipient may further be comprised.

Preferably, the dosage form of the pharmaceutical preparation comprises a liquid preparation and/or a lyophilized preparation; but not limited to these.

Preferably, the administration route of the pharmaceutical preparation comprises injection, oral administration, external application or administration via a combination of multiple routes; but not limited to these.

Preferably, a treatment method using the pharmaceutical preparation comprises one of or a combination of a radiotherapy, a chemotherapy, a myeloablative therapy, a CAR-T therapy, a CAR-NK therapy, an antibody-targeted therapy, an oncolytic virus therapy and a gene vaccine therapy; but not limited to these.

In the present invention, the polyethylene glycol derivative and IL-12 are coupled through covalent bonds to obtain the interleukin-12 with polyethylene glycol derivative modification introduced, so that the interleukin-12 has more advantages of becoming a potential drug product while maintaining the original biological activity of the interleukin-12. The advantages comprise, but are not limited to, increased molecular weight and hydration radius, improved *in vivo* stability, prolonged *in vivo* half-life, reduced immunogenicity, improved storage stability, storage capacity, and safe use as a liquid preparation, a frozen formulation, and a dried lyophilized formulation after several days. Polyethylene glycol

derivative modified interleukin-12 is able to balance the drug activity and drug toxicity of IL-12. Compared to natural IL-12, the polyethylene glycol derivative modified interleukin-12 can be administered in larger single doses and maintain effective blood concentration for a longer period of time without causing serous toxic side effects, which is beneficial for the continuity of treatment and improves the treatment effects of diseases. Therefore, polyethylene glycol-modified interleukin-12 has greater advantages over natural IL-12 for use in treatments such as anti-tumor, resistance to infection, and hemogram recovery. Compared to PEG-G-CSF and PEG-IL-2, polyethylene glycol derivative modified interleukin-12 has a larger molecular weight, which translates to a longer half-life. This allows for the maintenance of blood concentration for a longer period of time with each administration, thereby increasing the intervals between administrations, reducing the patient's discomfort, and saving time costs. Additionally, unlike PEG-G-CSF, which primarily functions to improve the hemogram recovery, and unlike PEG-IL-2, which primarily stimulates T cell proliferation and induces cytotoxic effects for anti-tumor purposes, the polyethylene glycol derivative modified interleukin-12 has a more extensive range of applications as it not only can promote the hemogram recovery but also can stimulate T cells to differentiate towards Th1, stimulate NK cells to exert more natural killer toxicity, and stimulate cells to express a series of cytokines. This overall enhances immunity, presenting significant potential in applications such as resistance to infection, anti-tumor, and hemogram recovery. The polyethylene glycol derivative modified interleukin-12 of the present invention can be widely used in one of or a combination of radiotherapy, chemotherapy, myeloablative therapy, CAR-T therapy, CAR-NK therapy, antibody-targeted therapy, oncolytic virus therapy and gene vaccine therapy, and has effects such as improving immunity, resistance to infection, anti-tumor and hemogram recovery.

Brief Description of the Drawings

Fig. 1 shows the UPLC chart of mPEG-pALD-20KD-IL-12 in Example 1 of the present invention.
Fig. 2 shows the molecular weight chart of mPEG-pALD-20KD-IL-12 in Example 1 of the present invention.
Fig. 3 shows the electrophoresis image of mPEG-pALD-20KD-IL-12 in Example 1 of the present invention.
Fig. 4 shows the UPLC chart of 2-arm PEG-CHO-20K-IL-12 in Example 2 of the present invention.
Fig. 5 shows the UPLC chart of mPEG-SS-20KD-IL-12 in Example 3 of the present invention.
Fig. 6 shows the molecular weight chart of mPEG-SS-20KD-IL-12 in Example 3 of the present invention.
Fig. 7 shows the UPLC chart of mPEG-MAL-20KD-IL-12 in Example 4 of the present invention.
Fig. 8 shows the molecular weight chart of mPEG-MAL-20KD-IL-12 in Example 4 of the present invention.
Fig. 9 shows the UPLC chromatographic comparison chart of mPEG-pALD-20KD-IL-12 in Example 1, mPEG-MAL-20KD-IL-12 in Example 4 and IL-12 of the present invention.
Fig. 10 shows the UPLC chart of 2-arm-PEG-MAL-20KD-IL-12 in Example 5 of the present invention.
Fig. 11 shows the UPLC chart of mPEG-SC-20KD-IL-12 in Example 6 of the present invention.
Fig. 12 shows the UPLC chart of mPEG-SC-5KD-IL-12 in Example 7 of the present invention.
Fig. 13 shows a diagram showing the counting results of cultured CD34+ cells in Example 15 of the present invention.
Fig. 14 shows an image of large red blood cell colonies (BFU-E) in the colony stimulation test in Example 15 of the present invention.
Fig. 15 shows an image of a single red blood cell colony (CFU-E) in the colony stimulation test in Example 15 of the present invention.
Fig. 16 shows an image of granulocyte/macrophage colonies (CFU-GM) in the colony stimulation test in Example 15 of the present invention.
Fig. 17 shows an image of a mixed colony of granulocyte/red blood cell/macrophage/megakaryocyte (CFU-GEMM) in the colony stimulation test in Example 15 of the present invention.
Fig. 18 shows a data summary chart of the colony stimulation test in Example 15 of the present invention.
Fig. 19 shows a data summary chart of long-term stimulation of NK92 cells by pegylated interleukin-12 in Example 19 of the present invention.
Fig. 20 shows a comparative chart of flow cytometry results of polyethylene glycol-modified interleukin-12 stimulating NK cells to kill Jurkat tumor cells in Example 20 of the present invention.
Fig. 21 shows a comparative chart of flow cytometry results of polyethylene glycol-modified interleukin-12 stimulating NK cells to kill K562 tumor cells in Example 20 of the present invention.
Fig. 22 shows a comparative chart of flow cytometry results of polyethylene glycol-modified interleukin-12 stimulating NK cells to kill SK-BR3 tumor cells in Example 20 of the present invention.
Fig. 23 shows a comparative chart of flow cytometry results of polyethylene glycol-modified interleukin-12 stimulating NK cells to kill NCI-N87 tumor cells in Example 20 of the present invention.
Fig. 24 shows a summary chart of flow cytometry results of polyethylene glycol-modified interleukin-12 stimulating NK cells to kill SK-BR3 tumor cells in Example 20 of the present invention.

Fig. 25 shows a summary chart of flow cytometry results of polyethylene glycol-modified interleukin-12 stimulating NK cells to kill NCI-N87 tumor cells in Example 20 of the present invention.

Fig. 26 shows a comparative chart of denaturation curves and aggregation curves of natural IL-12(S1), mPEG-MAL-20K-IL-12(S2) and mPEG-pALD-20K-IL-12(S3) in the experiment for detecting the stability of polyethylene glycol-modified interleukin-12 in Example 21 of the present invention.

Detailed Description of Embodiments

In order to understand the technical features, object and beneficial effects of the present invention more clearly, then the technical solution of the present invention is described in detail as below, but it cannot be construed as a limitation for the implementable scope of the present invention. Unless otherwise specified, the raw material components in the following examples are all commercially available in the art.

**Example 1**

This example provides a synthesis of monodisperse 20 KD monomethoxy polyethylene glycol propionaldehyde (mPEG-pALD-20K)-modified interleukin-12. The specific preparation method is as follows:

(1) 5 mg of interleukin-12 was taken and dissolved in a 0.05 M acetic acid/sodium acetate buffer with a pH of 5.0. The mixture was stirred at 20°C until dissolved to prepare a solution with a final concentration of 1.0 mg/mL of interleukin 12.

(2) 14.3 mg of monomethoxy polyethylene glycol propionaldehyde with a molecular weight of 20 KD (purchased from Xiamen Sinopeg Biotechnology Co., Ltd., model: 06020101412) was dissolved in pure water to prepare an aqueous solution of monomethoxy polyethylene glycol propionaldehyde; Then the aqueous solution was added dropwise into the interleukin-12 solution, where the molar ratio of the monomethoxy polyethylene glycol propionaldehyde to the interleukin-12 was 10 : 1.

(3) 0.135 mg of sodium cyanoborohydride (the molar ratio of sodium cyanoborohydride to interleukin-12 is 30 : 1) was dissolved in water to prepare a aqueous solution of sodium cyanoborohydride, which was then added to the interleukin-12 solution. The mixture was reacted under stirring at 20°C for 24 h, and the reaction was monitored by UPLC, as shown in Table 1 and Fig. 1.

Table 1:

| | Retention time (min) | Area (μV*s) | Height (μV) | Area% |
|---|---|---|---|---|
| 1 | 12.801 | 19562 | 2065 | 6.77 |
| 2 | 14.360 | 192740 | 12409 | 66.68 |
| 3 | 15.003 | 69885 | 5067 | 24.18 |
| 4 | 15.385 | 6856 | 612 | 2.37 |

It can be seen from Table 1 and Fig. 1 that: UPLC monitors the synthesis reaction. The peak corresponding to the retention time of 14.360 min is the single modification product, that is: monodisperse 20 KD monomethoxy polyethylene glycol propionaldehyde-modified interleukin-12 (named: mPEG-pALD-20KD-IL-12), with a yield of 66.68%.

(4) After the reaction was completed, the product was purified, specifically: first, the product was filtrated using a 30 KD cut-off ultrafiltration tube, and then purified through ion exchange chromatography to obtain purified monodispersed 20 KD monomethoxy polyethylene glycol propionaldehyde-modified interleukin-12.

The specific reaction flow for the preparation method of this example is as follows:

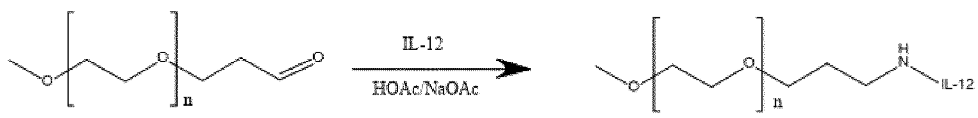


mPEG-pALD-20K

mPEG-pALD-20K-IL-12

The value of n was approximately 454.

In this example, selective site-specific coupling of mPEG-pALD-20KD on the N-terminal α-amino group of interleukin-12 was achieved by controlling the above reaction conditions.

The HLC-8420GPC&LenS3MALS detector was used to detect the molecular weight of the synthesized mPEG-pALD-20KD-IL-12. The experimental results are shown in Fig. 2. It can be seen from the molecular weight chart in Fig. 2 that the molecular weight of mPEG-pALD-20KD-IL-12 obtained through modification in this example was calculated to be approximately 90 KD. (Calculation method: $M_w^{-1}$ = KC/$R_{LALS}$; where $M_w^{-1}$ is the molecular weight calculated from the above formula, K is the coefficient of low-angle light scattering intensity and molecular weight, C is the solution concentration, $R_{LALS}$ is the low-angle light scattering intensity)

SDS-PAGE was used to detect the purity of purified mPEG-pALD-20KD-IL-12. The electrophoresis image is shown in Fig. 3. In Fig. 3, A1-A9 are the solutions eluted from the purification gradient collected at different times, where the single modification product mPEG-pALD-20KD-IL-12 of interest has an apparent molecular weight of about 133 KD, the purity of products A5-A7 are all above 95%, while A1-A4 and A8 and A9 are present at small amounts.

**Example 2**

This example provides the synthesis of monodisperse 20 KD two-arm polyethylene glycol propionaldehyde (2-arm PEG-CHO-20K)-modified interleukin-12. The specific preparation method is as follows:

(1) 5 mg of interleukin-12 was dissolved in an acetic acid buffer with a pH value of 5.0. The mixture was stirred at 20°C until dissolved to prepare an interleukin-12 solution with a final concentration of 1.0 mg/mL.

(2) 14.3 mg of two-arm polyethylene glycol propionaldehyde with a molecular weight of 20 KD (purchased from Xiamen Sinopeg Biotechnology Co., Ltd., model: 6022108312) was dissolved in pure water to prepare an aqueous solution of two-arm polyethylene glycol propionaldehyde; and then the aqueous solution was added dropwise to the interleukin-12 solution, where the molar ratio of the two-arm polyethylene glycol propionaldehyde to the interleukin-12 was 10 : 1.

(3) 0.135 mg of sodium cyanoborohydride (with the molar ratio of sodium cyanoborohydride to interleukin-12 of 30 : 1) was dissolved in water to prepare an aqueous solution of sodium cyanoborohydride, which was then added to the interleukin-12 solution, the mixture was reacted under stirring at 20°C for 24 h, and the reaction was monitored by UPLC, as shown in Table 2 and Fig. 4.

Table 2:

| | Retention time (min) | Area (μV*s) | Height (μV) | Area% |
|---|---|---|---|---|
| 1 | 11.138 | 425803 | 41879 | 23.95 |
| 2 | 12.409 | 971388 | 76846 | 54.64 |
| 3 | 12.940 | 380739 | 19275 | 21.41 |

It can be seen from Table 2 and Fig. 4 that: UPLC monitors the synthesis reaction. The peak corresponding to the retention time of 12.409 min is the single modification product, that is: two-arm polyethylene glycol propionaldehyde-modified interleukin-12 (named: 2-arm PEG-CHO-20K-IL-12), with a yield of 54.64%.

(4) After the reaction was completed, the product was purified, specifically: first, the product was filtrated using a 30 KD cut-off ultrafiltration tube, and then purified through ion exchange chromatography to obtain purified two-arm polyethylene glycol propionaldehyde-modified interleukin-12.

The specific reaction flow for the preparation method of this example is as follows:

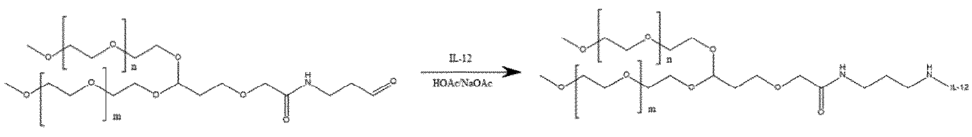


2-arm PEG-CHO -20K

2-arm PEG-CHO -20K-IL-12

The n or m value was approximately 227.

In this example, selective site-specific coupling of 2-arm PEG-CHO-20K on the N-terminal α-amino group of interleukin-12 was achieved by controlling the above reaction conditions.

It can be seen from the UPLC chromatogram that the target products of Example 1 and Example 2 have the same theoretical molecular weight, but the retention times are different. The retention time of Example 1 is 14.360 min, and the retention time of Example 2 is 12.409 min. The retention time of the two relative to IL-12 is also different, and

the relative retention time of Example 2 is smaller. This is because the product molecule in Example 2 is two-armed and has a larger apparent molecular weight, and the packing of the chromatographic column used has a pore size of 450 Å and has certain characteristics of size exclusion chromatography, so the product of Example 2 with a larger apparent molecular weight has a shorter retention time in the column than Example 1 and comes out earlier.

Therefore, under the same molecular weight, multi-arm PEG modification brings better modification effect than single-arm PEG modification. This is mainly because multi-arm PEG has a larger apparent molecular weight, a greater steric hindrance, and at the same time covers a smaller area of IL-12 and can retain more IL-12 activity.

**Example 3**

This example provides a synthesis of monodisperse 20 KD monomethoxy polyethylene glycol succinimide succinate (mPEG-SS-20KD)-modified interleukin-12. The specific preparation method is as follows:

(1) 5 mg of interleukin-12 was taken and dissolved in a phosphate buffer with a pH value of 7.4. The mixture was stirred at 20°C until dissolved to prepare a solution with a final concentration of 0.8 mg/mL of interleukin 12.
(2) 7.85 mg of monomethoxy polyethylene glycol succinimide succinate with a molecular weight of 20 KD (purchased from Xiamen Sinopeg Biotechnology Co., Ltd., model: 06020102512) was dissolved in pure water to prepare an aqueous solution of monomethoxy polyethylene glycol succinimide succinate; Then the aqueous solution was added dropwise into the interleukin-12 solution, where the molar ratio of the monomethoxy polyethylene glycol succinimide succinate to the interleukin-12 was 5.5 : 1. The mixture was reacted under stirring at 8°C for 3 h, and the reaction was monitored by UPLC, as shown in Table 3 and Fig. 5.

Table 3:

| | Retention time (min) | Area (μV*s) | Height (μV) | Area% |
|---|---|---|---|---|
| 1 | 12.408 | 73836 | 5431 | 28.29 |
| 2 | 13.987 | 108000 | 5332 | 41.38 |
| 3 | 14.545 | 60592 | 3762 | 23.21 |
| 4 | 14.888 | 18590 | 1251 | 7.12 |

It can be seen from Table 3 and Fig. 5 that: UPLC monitors the synthesis reaction. The peak corresponding to the retention time of 13.987 min is the single modification product, that is: monomethoxy polyethylene glycol succinimide succinate-modified interleukin-12 (named: mPEG-SS-20KD-IL-12), with a yield of 41.38%.

(3) Purification of the product after the reaction was completed: first, the product was filtrated using a 30 KD cut-off ultrafiltration tube, and then purified through ion exchange chromatography to obtain purified monomethoxy polyethylene glycol succinimide succinate-modified interleukin-12.

The specific reaction flow for the preparation method of this example is as follows:

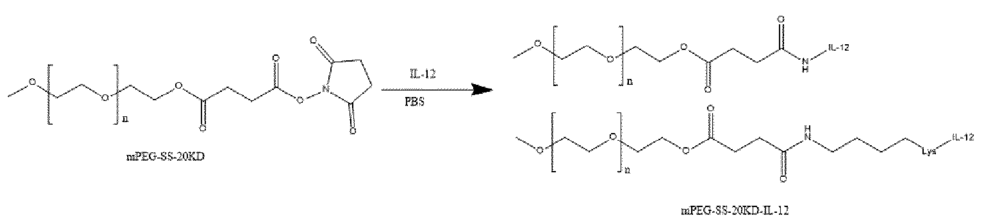


The value of n was approximately 454.

In this example, selective site-specific coupling of mPEG-SS-20KD on the N-terminal α-amino group or the ε-amino group of the lysine side chains of interleukin-12 was achieved by controlling the above reaction conditions. The polyethylene glycol active ester derivative modified interleukin-12 of this example has the characteristic of reversible modification, and during use, it enables polyethylene glycol to separate from IL-12 again through a hydrolysis reaction, thereby allowing IL-12 to retain more complete biological activity.

The HLC-8420GPC&LenS3MALS detector was used to detect the molecular weight of the synthesized mPEG-SS-20KD-IL-12. The experimental results are shown in Fig. 6. It can be seen from the molecular weight chart in Fig. 6 that the molecular weight of mPEG-SS-20KD-IL-12 obtained through modification in this example was calculated to be

approximately 90 KD.

**Example 4**

This example provides a synthesis of monodisperse 20 KD monomethoxy polyethylene glycol maleimide (mPEG-MAL-20KD)-modified interleukin-12. The specific preparation method is as follows:
(1) 25 mg of interleukin-12 was taken and dissolved in a phosphate buffer with a pH value of 7.0. The mixture was stirred at 20°C until dissolved to prepare a solution with a final concentration of 1.0 mg/mL of interleukin 12.
(2) 71 mg of monomethoxy polyethylene glycol maleimide with a molecular weight of 20 KD (purchased from Xiamen Sinopeg Biotechnology Co., Ltd., model: 06020101912) was dissolved in pure water to prepare an aqueous solution of monomethoxy polyethylene glycol maleimide;
Then the aqueous solution was added dropwise into the interleukin-12 solution, where the molar ratio of the monomethoxy polyethylene glycol maleimide to the interleukin-12 was 10 : 1. The mixture was reacted under stirring at 20°C for 24 h, and the reaction was monitored by UPLC, as shown in Table 4 and Fig. 7.

Table 4:

| | Retention time (min) | Area (μV*s) | Height (μV) | Area% |
|---|---|---|---|---|
| 1 | 12.770 | 60303 | 5361 | 12.12 |
| 2 | 14.245 | 361271 | 23974 | 72.62 |
| 3 | 15.592 | 75908 | 7345 | 15.26 |

It can be seen from Table 4 and Fig. 7 that: UPLC monitors the synthesis reaction. The peak corresponding to the retention time of 14.245 min is the single modification product, that is: monomethoxy polyethylene glycol maleimide-modified interleukin-12 (named: mPEG-MAL-20KD-IL-12), with a yield of 72.62%.

(3) After the reaction was completed, the product was purified, specifically: first, the product was filtrated using a 30 KD cut-off ultrafiltration tube, and then purified through ion exchange chromatography to obtain purified monomethoxy polyethylene glycol maleimide-modified interleukin-12.

The specific reaction flow for the preparation method of this example is as follows:

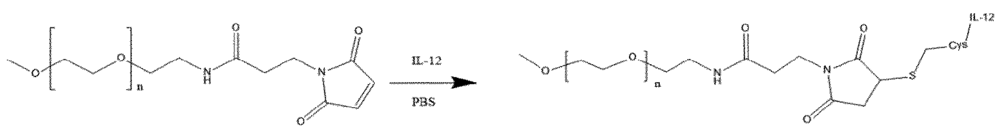


The value of n was approximately 454.

In this example, selective site-specific coupling of mPEG-MAL-20KD on the free sulfhydryl group of cysteine at the position C252 of the P40 subunit of interleukin-12 was achieved by controlling the above reaction conditions.

The HLC-8420GPC&LenS3MALS detector was used to detect the molecular weight of the synthesized mPEG-MAL-20KD-IL-12. The experimental results are shown in Fig. 8. It can be seen from the molecular weight chart in Fig. 8 that the molecular weight of mPEG-MAL-20KD-IL-12 obtained through modification in this example was calculated to be approximately 96 KD.

IL-12, mPEG-pALD-20KD-IL-12 prepared in Example 1 and mPEG-MAL-20KD-IL-12 prepared in Example 4 were subjected to UPLC examination after reduction with mercaptoethanol. The UPLC chromatogram is shown in Fig. 9. It can be seen from the comparison in Fig. 9 that: For the PEGs in mPEG-pALD-20KD-IL-12, some of the PEGs are attached to the P35 subunit of IL-12 and some of the PEGs are attached to the P40 subunit of IL-12, and most of the PEGs are attached to the P35 subunit; all of the PEGs of mPEG-MAL-20KD-IL-12 are attached to the P40 subunit, and because there is only a free cysteine at position 252 of the P40 subunit, so it is proved that the modification site of the PEG of mPEG-MAL-20KD-IL-12 is C252 of P40.

**Example 5**

This example provides a synthesis of monodisperse 20 KD branched monomethoxy polyethylene glycol maleimide (2-arm-PEG-MAL-20KD)-modified interleukin-12. The specific preparation method is as follows:
(1) 25 mg of interleukin-12 was taken and dissolved in a phosphate buffer with a pH value of 7.0. The mixture was stirred

at 20°C until dissolved to prepare a solution with a final concentration of 1.0 mg/mL of interleukin 12.
(2) 71 mg of branched monomethoxy polyethylene glycol maleimide with a molecular weight of 20 KD (purchased from Xiamen Sinopeg Biotechnology Co., Ltd., model: 06022101812) was dissolved in pure water to prepare an aqueous solution of branched monomethoxy polyethylene glycol maleimide; Then the aqueous solution was added dropwise into the interleukin-12 solution, where the molar ratio of the branched monomethoxy polyethylene glycol maleimide to the interleukin-12 was 10 : 1. The mixture was reacted under stirring at 20°C for 24 h, and the reaction was monitored by UPLC, as shown in Table 5 and Fig. 10.

Table 5:

| | Retention time (min) | Area (μV*s) | Height (μV) | Area% |
|---|---|---|---|---|
| 1 | 11.119 | 493912 | 47265 | 19.81 |
| 2 | 11.705 | 65360 | 3768 | 2.62 |
| 3 | 12.273 | 1498860 | 103528 | 60.11 |
| 4 | 13.530 | 435542 | 33879 | 17.47 |

It can be seen from Table 5 and Fig. 10 that: UPLC monitors the synthesis reaction. The peak corresponding to the retention time of 12.273 min is the single modification product, that is: branched monomethoxy polyethylene glycol maleimide-modified interleukin-12 (named: 2-arm-PEG-MAL-20KD-IL-12), with a yield of 60.11%.
(3) After the reaction was completed, the product was purified, specifically: the product was filtrated using a 30 KD cut-off ultrafiltration tube, and then purified through ion exchange chromatography to obtain purified branched monomethoxy polyethylene glycol maleimide-modified interleukin-12.
The specific reaction flow for the preparation method of this example is as follows:

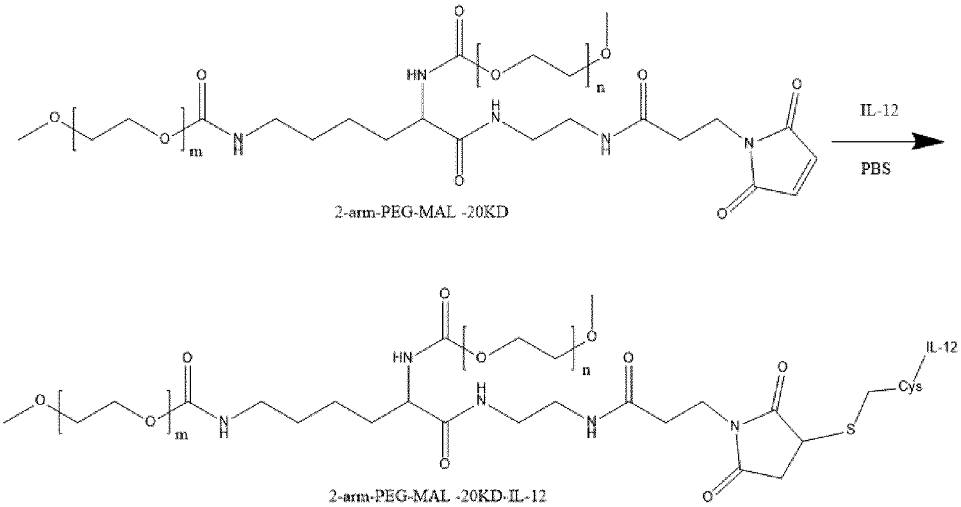

2-arm-PEG-MAL -20KD

2-arm-PEG-MAL -20KD-IL-12

The value of n or m was approximately 227.
In this example, selective site-specific coupling of 2-arm-PEG-MAL-20KD on the free sulfhydryl group of cysteine at the position C252 of the P40 subunit of interleukin-12 was achieved by controlling the above reaction conditions.
It can be seen from the UPLC chromatogram that the target products of Example 4 and Example 5 have the same theoretical molecular weight, but the retention times are different. The retention time of Example 4 is 14.245 min, and the retention time of Example 5 is 12.273 min. The retention time of the two relative to IL-12 is also different, and the relative retention time of Example 5 is smaller. This is because the product molecule in Example 5 is two-armed and has a larger apparent molecular weight, and the packing of the chromatographic column used has a pore size of 450 Å and has certain characteristics of size exclusion chromatography, so the product of Example 5 with a larger apparent molecular weight has a shorter retention time in the column than Example 4 and comes out earlier.
Therefore, under the same molecular weight, multi-arm PEG brings better modification effect than single-arm PEG. This is mainly because multi-arm PEG has a larger apparent molecular weight, a greater steric hindrance, and at the same time covers a smaller area of IL-12 and can retain more IL-12 activity.

**Example 6**

This example provides a synthesis of monodisperse 20 KD monomethoxy polyethylene glycol succinimide carbonate (mPEG-SC-20KD)-modified interleukin-12. The specific preparation method is as follows:

(1) 5 mg of interleukin-12 was taken and dissolved in a phosphate buffer with a pH value of 8.0. The mixture was stirred at 8°C until dissolved to prepare a solution with a final concentration of 1.0 mg/mL of interleukin 12.
(2) 28.5 mg of monomethoxy polyethylene glycol succinimide carbonate with a molecular weight of 20 KD (purchased from Xiamen Sinopeg Biotechnology Co., Ltd., model: 06020102012) was dissolved in pure water to prepare an aqueous solution of monomethoxy polyethylene glycol succinimide carbonate; Then the aqueous solution was added dropwise into the interleukin-12 solution, where the molar ratio of the monomethoxy polyethylene glycol succinimide carbonate to the interleukin-12 was 20 : 1. The mixture was reacted under stirring at 8°C for 4 h, and the reaction was monitored by UPLC, as shown in Table 6 and Fig. 11.

Table 6:

| | Retention time (min) | Area (μV*s) | Height (μV) | Area% |
|---|---|---|---|---|
| 1 | 15.788 | 2248 | 377 | 0.98 |
| 2 | 16.255 | 49666 | 3428 | 21.65 |
| 3 | 16.558 | 177520 | 7860 | 77.37 |

It can be seen from Table 6 and Fig. 11 that: UPLC monitors the synthesis reaction. The peak corresponding to the retention time of 16.558 min is the single modification product, that is: monomethoxy polyethylene glycol succinimide carbonate-modified interleukin-12 (named: mPEG-SC-20KD -IL-12), with a yield of 77.37%.

(3) Purification of the product after the reaction was completed: first, the product was filtrated using a 30 KD cut-off ultrafiltration tube, and then purified through ion exchange chromatography to obtain purified monomethoxy polyethylene glycol succinimide carbonate-modified interleukin-12.

The specific reaction flow for the preparation method of this example is as follows:

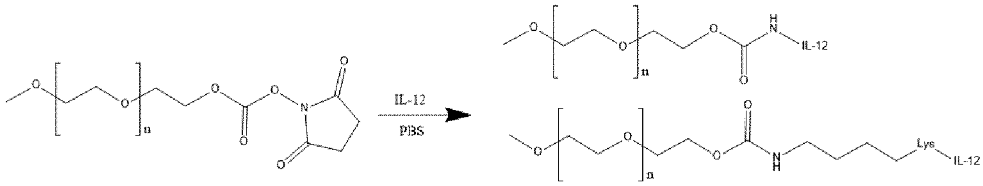


The value of n was approximately 454.

In this example, the saturated and reversible modification of interleukin-12 by mPEG-SC-20KD was achieved by controlling the above reaction conditions. Selective site-specific coupling modification on the terminal α-amino group or the ε-amino group of the lysine side chains of interleukin-12 was achieved. The polyethylene glycol active ester derivative modified interleukin-12 of this example has the characteristic of reversible modification, and during use, it enables polyethylene glycol to separate from IL-12 again through a hydrolysis reaction, thereby allowing IL-12 to retain more complete biological activity.

**Example 7**

This example provides a synthesis of monodisperse 5 KD monomethoxy polyethylene glycol succinimide carbonate (mPEG-SC-5KD)-modified interleukin-12. The specific preparation method is as follows:

(1) 5 mg of interleukin-12 was taken and dissolved in a phosphate buffer with a pH value of 8.0. The mixture was stirred at 8°C until dissolved to prepare a solution with a final concentration of 1.0 mg/mL of interleukin 12.
(2) 7.1 mg of monomethoxy polyethylene glycol succinimide carbonate with a molecular weight of 5 KD (purchased from Xiamen Sinopeg Biotechnology Co., Ltd., model: 06020102006) was dissolved in pure water to prepare an aqueous solution of monomethoxy polyethylene glycol succinimide carbonate; Then the aqueous solution was added

dropwise into the interleukin-12 solution, where the molar ratio of the monomethoxy polyethylene glycol succinimide carbonate to the interleukin-12 was 20 : 1. The mixture was reacted under stirring at 8°C for 4 h, and the reaction was monitored by UPLC, as shown in Table 7 and Fig. 12.

Table 7:

| | Retention time (min) | Area (μV*s) | Height (μV) | Area% |
|---|---|---|---|---|
| 1 | 13.961 | 478526 | 12977 | 100.00 |

It can be seen from Table 7 and Fig. 12 that: UPLC monitors the synthesis reaction. The peak corresponding to the retention time of 13.961 min is the single modification product, that is: monomethoxy polyethylene glycol succinimide carbonate-modified interleukin-12 (named: mPEG-SC-5KD-IL-12), with a yield of 100%.

(3) After the reaction was completed, the product was purified, specifically: first, the product was filtrated using a 30 KD cut-off ultrafiltration tube, and then purified through ion exchange chromatography to obtain purified monomethoxy polyethylene glycol succinimide carbonate-modified interleukin-12.

The specific reaction flow for the preparation method of this example is as follows:

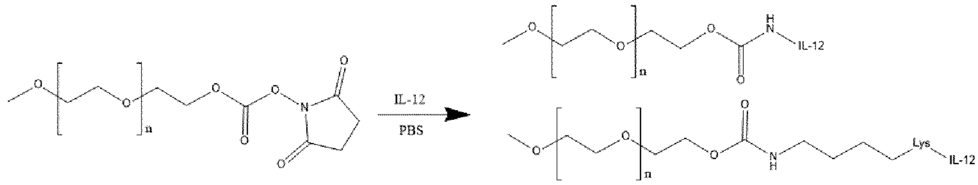


The value of n was approximately 113.

In this example, selective site-specific coupling of mPEG-SC-5KD on the terminal α-amino group or the ε-amino group of the lysine side chains of interleukin-12 was achieved by controlling the above reaction conditions. The polyethylene glycol active ester derivative modified interleukin-12 of this example has the characteristic of reversible modification, and during use, it enables polyethylene glycol to separate from IL-12 again through a hydrolysis reaction, thereby allowing IL-12 to retain more complete biological activity.

**Example 8**

This example provides a synthesis of monodisperse 20 KD monomethoxy polyethylene glycol vinyl sulfone (mPEG-VS-20KD)-modified interleukin-12. The specific preparation method is as follows:

(1) 25 mg of interleukin-12 was taken and dissolved in a phosphate buffer with a pH value of 8.0. The mixture was stirred at 8°C until dissolved to prepare a solution with a final concentration of 1.0 mg/mL of interleukin 12.
(2) 71 mg of monomethoxy polyethylene glycol vinyl sulfone with a molecular weight of 20 KD (purchased from Xiamen Sinopeg Biotechnology Co., Ltd., model: 06022105012) was dissolved in pure water to prepare an aqueous solution of monomethoxy polyethylene glycol vinyl sulfone; Then the aqueous solution was added dropwise into the interleukin-12 solution, where the molar ratio of the monomethoxy polyethylene glycol vinyl sulfone to the interleukin-12 was 10 :

1. The mixture was reacted under stirring at 20°C for 24 h, and the reaction was monitored by UPLC.

(3) After the reaction was completed, the product was purified, specifically: first, the product was filtrated using a 30 KD cut-off ultrafiltration tube, and then purified through ion exchange chromatography to obtain purified monomethoxy polyethylene glycol vinyl sulfone-modified interleukin-12 (mPEG-VS-20KD-IL-12).

The specific reaction flow for the preparation method of this example is as follows:

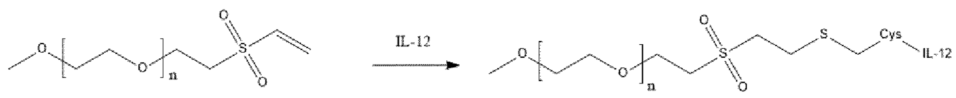


mPEG-VS-20K mPEG-VS-20K-IL-12

The value of n was approximately 454.

**Example 9**

This example provides a synthesis of monodisperse 20 KD monomethoxy polyethylene glycol iodoacetamide (mPEG-IA-20KD)-modified interleukin-12. The specific preparation method is as follows:

(1) 25 mg of interleukin-12 was taken and dissolved in a phosphate buffer with a pH value of 7.0. The mixture was stirred at 20°C until dissolved to prepare a solution with a final concentration of 1.0 mg/mL of interleukin 12.
(2) 107 mg of monomethoxy polyethylene glycol iodoacetamide with a molecular weight of 20 KD (purchased from Shanghai Toyong Biol Co., Ltd, model: P001019-20000) was dissolved in pure water to prepare an aqueous solution of monomethoxy polyethylene glycol iodoacetamide; Then the aqueous solution was added dropwise into the interleukin-12 solution, where the molar ratio of the monomethoxy polyethylene glycol iodoacetamide to the interleukin-12 was 15 : 1. The mixture was reacted under stirring at 20°C for 24 h, and the reaction was monitored by UPLC.
(3) After the reaction was completed, the product was purified, specifically: first, the product was filtrated using a 30 KD cut-off ultrafiltration tube, and then purified through ion exchange chromatography to obtain purified monomethoxy polyethylene glycol iodoacetamide-modified interleukin-12 (mPEG-IA-20KD-IL-12).

The specific reaction flow for the preparation method of this example is as follows:

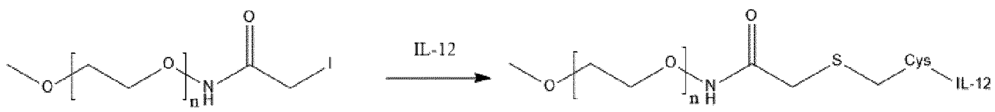


mPEG-IA-20K mPEG-IA-20K-IL-12

The value of n was approximately 454.

**Example 10**

This example provides a synthesis of monodisperse 20 KD monomethoxy polyethylene glycol dithioisopyridine (mPEG-OPSS-20KD)-modified interleukin-12. The specific preparation method is as follows:

(1) 25 mg of interleukin-12 was taken and dissolved in a phosphate buffer with a pH value of 6.0. The mixture was stirred at 20°C until dissolved to prepare a solution with a final concentration of 1.0 mg/mL of interleukin 12.
(2) 35 mg of monomethoxy polyethylene glycol dithioisopyridine with a molecular weight of 20 KD (purchased from Guangzhou Tanshui Technology Co., Ltd., model: 80010121-20000) was dissolved in pure water to prepare an aqueous solution of monomethoxy polyethylene glycol dithioisopyridine; Then the aqueous solution was added dropwise into the interleukin-12 solution, where the molar ratio of the monomethoxy polyethylene glycol dithioisopyridine to the interleukin-12 was 5 : 1. The mixture was reacted under stirring at 20°C for 24 h, and the reaction was monitored by UPLC.
(3) After the reaction was completed, the product was purified, specifically: first, the product was filtrated using a 30 KD cut-off ultrafiltration tube, and then purified through ion exchange chromatography to obtain purified monomethoxy polyethylene glycol dithioisopyridine-modified interleukin-12 (mPEG-OPSS-20KD-IL-12).

The specific reaction flow diagram for the preparation method of this example is as follows:

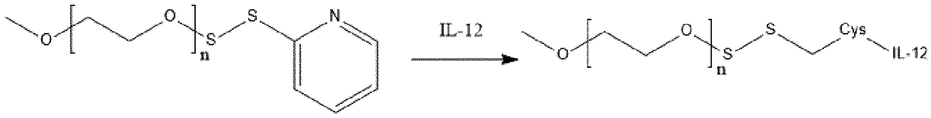


mPEG-OPSS-20K

mPEG-OPSS-20K-IL-12

The value of n was approximately 454.

### Example 11

This example provides a synthesis of monodisperse 20 KD monomethoxy polyethylene glycol acetic acid (mPEG-AA-20KD)-modified interleukin-12. The specific preparation method is as follows:

(1) 10 mg of interleukin-12 was taken and dissolved in a phosphate buffer with a pH value of 7.0. The mixture was stirred at 20°C until dissolved to prepare a solution with a final concentration of 1.0 mg/mL of interleukin 12.
(2) 15.7 mg of monomethoxy polyethylene glycol acetic acid with a molecular weight of 20 KD (purchased from Guangzhou Tanshui Technology Co., Ltd., model: 80010103-20000) was dissolved in pure water to prepare an aqueous solution of monomethoxy polyethylene glycol acetic acid; Then the aqueous solution was added dropwise into the interleukin-12 solution, and then 4.5 mg of the condensing agent EDC (the molar ratio of EDC to interleukin-12 was 200 : 1) was added, the mixture was reacted under stirring at 10°C for 24 h, where the molar ratio of the monomethoxy polyethylene glycol acetic acid to the interleukin-12 was 5.5 : 1. The reaction was monitored by UPLC.
(3) After the reaction was completed, the product was purified, specifically: first, the product was filtrated using a 30 KD cut-off ultrafiltration tube, and then purified through ion exchange chromatography to obtain purified monomethoxy polyethylene glycol acetic acid-modified interleukin-12 (mPEG-AA-20KD-IL-12).

The specific reaction flow diagram for the preparation method of this example is as follows:

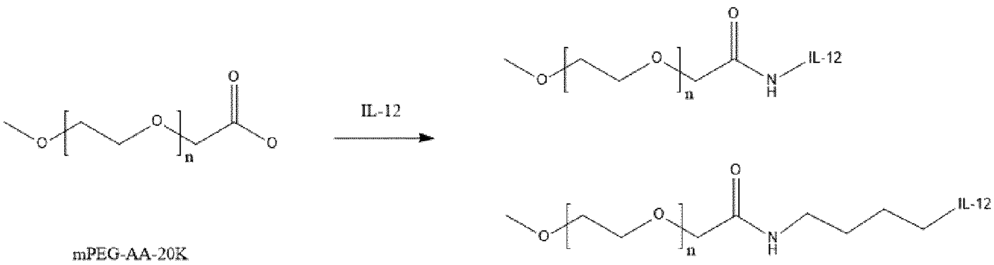


mPEG-AA-20K

mPEG-AA-20K-IL-12

The value of n was approximately 454.

### Example 12

Through comparative experiment of IFN-y production of NK-92 cells stimulated by mPEG-pALD-20KD-IL-12 (Example 1), mPEG- MAL-20KD-IL-12 (Example 4), mPEG-SS-20KD-IL-12 (Example 3) and IL-12 using IFN-y detection ELISA kit, the difference in activity and the degree of toxicity reduction between mPEG-pALD-20KD-IL-12, mPEG-MAL-20KD-IL-12, mPEG-SS-20KD-IL-12 and IL-12 were evaluated. The specific method was as follows:

(1) The cultured NK-92 cells were taken and their passage numbers recorded. The cell state was observed under a microscope, and if the cell state was good, the cells were collected in a sterile 15 ml or 50 ml centrifuge tube. The centrifuge tube was centrifuged at 600 rpm for 5 min, the supernatant was discarded and then the cells were resuspended in 5 ml of fresh complete medium and counted.
(2) Based on the cell count results, fresh complete culture medium was used to dilute the cell suspension to a cell suspension of $2 \times 10^5$ cells/ml, and then the cell suspension was added to a 96-well plate at 100 $\mu$l per well.
(3) The sample was filtered and sterilized, and gradiently diluted with NK-92 complete culture medium so that the final concentration was: 1000-0.46 U/ml. The diluted sample was added to a 96-well plate already containing 100 $\mu$l of cell suspension. The final culture system was 200 $\mu$l. The final cell density was $1 \times 10^5$ cells/ml. The cells were stimulated for (24 $\pm$ 2) h.

(4) After stimulation, the 96-well plate was centrifuged at 1000 rpm for 5 min. The supernatant was then diluted 60-fold with the standard dilution buffer (1X Dilution buffer R) provided in the ELISA Kit. Specifically, 5 μl of the supernatant was pipetted using an 10 μl 8-channel micropipette and added to 295 μl of the standard dilution buffer. The mixture was then subjected to high-speed agitation for 5 minutes using a plate washer. Then 100 μ! of the diluted supernatant was taken and added into the well of the ELISA plate of the IFN-y detection kit, and 50 μ! of biotinylated antibody was add and mixed thoroughly. The plate was then sealed with an adhesive film and incubated at room temperature (18-25°C) for 120 min. After incubation, the plate was washed four times with the wash buffer and then patted dry.

(5) Then 100 μ! of streptavidin-HRP was added and the mixture was incubated at room temperature (18-25°C) for 20 min. After incubation, the plate was washed four times with the wash buffer and then patted dry.

(6) 100 μl of TMB was added to each well and the mixture was incubated at room temperature (18-25°C) in the dark for 5-30 min. The liquid in the well gradually turned blue. After incubation at room temperature, 100 μl of a Stop solution was added to each well to stop the reaction, and a SpectraMax M2 microplate reader was used to detect the OD value at 450 nm.

For result processing, GraphPad Prism6 software was used to process the data. The OD value was used as the ordinate and the dilution factor was used as the abscissa. Four parameters were used for fitting to obtain the activity curve, Top value, Bottom value and $R^2$ value, the test standard required $R^2$ value ≧ 0.95. The activity of the test product was calculated according to the following formula:

$$\text{Biological activity of test product (U/ml)} = P_r \times \frac{D_s \times E_s}{D_r \times E_r}$$

Among them, Pr is the biological activity of the standard product, that is, the biological activity of the WHO IL-12 standard product = 1 × $10^4$ U/ml; Ds is the pre-dilution factor of the test product; Dr is the pre-dilution factor of the standard product; Es is the dilution factor of the test product equivalent to the median effective dose of the standard product; Er is the dilution factor of the median effective dose of the standard product. Specific activity of the test product (U/mg) = titer of the test product (U/ml)/concentration of the test product (mg/ml).

The experimental results are shown in Table 8:

Table 8:

| Name | Sample titer ($10^6$ U/mL) | Sample specific activity ($10^6$ U/mg) | Protein content (mg/mL) | Fold decrease (relative to the same batch of stock solution) |
|---|---|---|---|---|
| IL-12 stock solution | 10.55 | 10.4 | 1.01 | |
| mPEG-pALD-20KD-IL-12 | 0.08 | 0.27 | 0.30 | 8.0 |
| mPEG- MAL-20KD-IL-12 | 0.07 | 0.19 | 0.39 | 20.3 |
| mPEG-SS-20KD-IL-12 | 0.61 | 0.89 | 0.69 | 11.70 |

It can be seen from the experimental results in Table 8 above that: Compared with the IL-12 standard, the activity of the product mPEG-pALD-20KD-IL-12 obtained in Example 1 decreased by 8.0-fold, and the activity of the product mPEG-MAL-20KD-IL-12 obtained in Example 4 decreased by 20.3-fold, and the activity of the product mPEG-SS-20KD-IL-12 with single modification obtained in Example 3 decreased by 11.70-fold. The decrease of activity means that the dose of polyethylene glycol-modified interleukin-12 was larger than that of IL-12 in order to produce the same amount of IFN-y. On the other hand, polyethylene glycol-modified interleukin-12 exhibited lower side effects at the same dose, which facilitates the administration of a larger dose in a single time and maintains stable drug concentration in the blood for a longer period of time without causing major side effects. The dose of polyethylene glycol-modified interleukin-12 used in subsequent pharmacodynamic tests was determined with reference to the experimental results obtained in Example 12.

**Example 13**

This example provided an analysis experiment on the effect of the drug action duration of mPEG-pALD-20KD-IL-12 prepared in Example 1 in normal rats. Through the comparative experiment of the action duration of mPEG-pALD-20KD-IL-12 prepared in Example 1 and IL-12 in normal rats, it was confirmed that PEG modification of long-acting interleukin-12 could prolong the drug action duration.

Eight-week-old normal rats were divided into a control group (vehicle), a group administered with mPEG-pALD-20KD-IL-12, and a group administered with interleukin-12. For 3 rats in each group, test substances were administered to normal rats subcutaneously once, and whole blood samples were collected from their tail veins at 1, 4, 8, 24, 48, and 72 hours respectively. The whole blood samples were added into each 1.5 mL microtube, and the microtubes were centrifuged at 5000 rpm for 10 min at room temperature. Each serum was separated and stored at -20°C, respectively. The polyethylene glycol-modified interleukin-12 or simple interleukin-12 contained in the serum of each group was quantified by ELISA. The ELISA assay was performed in the following manner: the serum collected each time and anti-human interleukin-12-HRP were added to a plate coated with interleukin-12 monoclonal antibody at the same time. The reaction was carried out at room temperature for 1 h. TMB reagent was used to develop the color. The absorbance was measured at a wavelength of 450 nm, respectively.

The results showed that: the group administered long-acting interleukin-12 containing a 20 kDa PEG linker showed an increase in AUC compared to the group administered interleukin-12.

**Example 14**

This example provided an experiment on the resistance effect to infection of polyethylene glycol derivative modified interleukin-12; The unique biological activity of IL-12 enables it to promote the differentiation and maturation of Thl cells, and can stimulate T cells and NK cells to secrete a large number of IFN-$\gamma$, TNF-$\alpha$ and GM-CSF, and activate the killing effect of T cells and NK cells. IL-12 plays an extremely important role in the body's early nonspecific immunity and antigen-specific adaptive immunity. Therefore, IL-12 plays a significant role in resistance to bacterial infection, resistance to viral infection, resistance to parasitic infection, *etc.* Polyethylene glycol derivative modified interleukin-12 was used in the experiment on resistance effect to infection. The unique biological activity of IL-12 enables it to promote the differentiation and maturation of Thl cells, stimulate human peripheral blood mononuclear cells, and kill target cells infected with herpes simplex virus type I to evaluate the resistance effect to infection of PEG modified interleukin-12. The detailed experimental steps are as follows:

(1) PBMC cells were obtained and cultured in RPMI 1640 medium for 10 days.

(2) Then the cells were inoculated at $2 \times 10^6$/well into a 24-well plate, and different concentrations of mPEG-MAL-20KD-IL-12 prepared in Example 4 (200 ng/mL, 20 pg/mL, and 0.2 pg/mL) were added, while IL-12 (10 ng/mL, 1 pg/mL, and 0.01 pg/mL) was set as a control, and the same volume of buffer was added as a blank control, and the cells were continuously cultured for 7 days.

(3) At the same time, L929 cells were cultured with RPMI 1640 medium. L929 cells were labeled with 5 $\mu$mol/L Calcein AM in a 37°C water bath for 30 min. Then the medium was changed twice to remove the residual Calcein AM. Virus solution was added, and the culture was continued for 2 h to obtain target cells.

(4) Then PBMC was collected, inoculated into a 96-well plate at a ratio of 1/10 (*i.e.*, $1 \times 10^5$ target cells and $1 \times 10^6$ effector cells), and incubated in a 37°C 5% $CO_2$ incubator for 24 h. At the same time, the target cells cultured alone was set as a blank control, and the lysis with triton lysis solution was set as the maximum killing well. Centrifugation was performed at 3000 r/min for 5 min and the supernatant was transferred to a new well.

(5) Fluorescence detection: A fluorescence measuring instrument was used to detect the fluorescence value (FI) of the supernatant. The excitation light was 485 nm and the emission light was 538 nm. The differences in the killing effect of PBMC on target cells under the stimulation of various concentrations of PEGylated interleukin-12 and IL-12 were evaluated based on the detection values to illustrate the role of polyethylene glycol-modified interleukin-12 in resistance to infection.

**Example 15**

This example provided an experiment on the effect of polyethylene glycol derivative modified interleukin-12 on hemogram recovery; Myeloablative therapy is often used in the treatment of hematological cancers. As the therapy progresses, various blood cells undergo a process of decline and then gradual recovery. In the treatment of cancers, conventional chemotherapy and radiotherapy can also cause abnormal decreases in various cells in the blood, including decrease of neutrophils, decrease of platelets and decrease of lymphocytes, and are easy to bring serious side effects, including spontaneous bleeding caused by decrease of platelets, usually accompanied by fatigue and sometimes depression. The decrease in white blood cells, neutrophils, and lymphocytes can also lead to a decrease in the body's own immunity, making it susceptible to infection and other complications. Acute radiation syndrome, also known as radiation poisoning or radiation sickness (ARS), has an onset and type of symptoms that depend on the patient's radiation exposure. A low dose of radiation can cause effects on the digestive system, such as nausea, vomiting, and symptoms associated with a decrease in blood indices such as infection and bleeding. A high dose of radiation can cause symptoms of neurological damage and rapid death. In the hematopoietic subsyndrome of ARS (HSARS), rapid bone marrow clearance caused by large amounts of harmful rays reduces the number of various blood cells. Depending on the dose of radiation exposed, symptoms such as infection and bleeding generally occur within 2 weeks to 2 months, and eventually leading to death. The general treatment for acute radiation syndrome is blood transfusion and antibiotics.

IL-12 can stimulate hematopoietic function and promote hemogram recovery. IL-12 induces the production of IFN-y through the Stat4 pathway, and then IFN-y acts on hematopoietic stem cells. However, studies have shown that IFN-y plays a dual role (stimulatory/inhibitory role) in the hematopoietic process. IL-12 toxicity is associated with overproduction of IFN-y, suggesting that the effects of IL-12 on blood recovery may be dose-related: low-dose IL-12 can promote hematopoiesis, while high-dose IL-12 can inhibit hematopoiesis by inducing excessive IFN-y production and toxic reactions.

In this example, PEG-modified IL-12 is used to balance the drug activity and drug toxicity of IL-12, which can not only stimulate cells to produce an appropriate amount of IFN-y, but also make IL-12 suitable for hemogram recovery. CD34+ cells are hematopoietic stem cells. We evaluated the effect of polyethylene glycol-modified IL-12 on the hemogram recovery by comparing the proliferation and differentiation of CD34+ cells stimulated *in vitro* with mPEG-MAL-20K-IL-12, mPEG-pALD-20K-IL-12 and IL-12. The specific experimental steps are as follows:

(1) Different culture media were formulated. First, 100 ng/ml SCF, 20 ng/ml TPO, and 100 ng/ml Flt-3L were added to stemline II culture medium as the basic culture medium. Then, 6 $\times$ 10 ml aliquots of this culture medium were separated and supplemented with varying concentrations of mPEG-MAL-20KD-IL-12 prepared in Example 4 (2 ug/ml, 200 ng/ml, and 20 ng/ml), and mPEG-pALD-20KD-IL-12 prepared in Example 1 (800 ng/ml, 80 ng/ml, and 8 ng/ml) or different concentrations of IL-12 (100 ng/ml, 10 ng/ml, and 1 ng/ml);

(2) 1 $\times$ $10^4$ CD34+ cells were inoculated in 24-well plates containing the various culture media. Three groups of parallel wells were set up for each culture medium, and a blank control group was set. While refreshing the culture medium, the PEGylated IL-12 or IL-12 was updated accordingly, and culture was performed continuously for 10 days. Every two days, samples were taken and the number of cells was counted.

(3) At the same time, a colony stimulation test was performed. The human hematopoietic stem cell colony culture medium was placed in a 4°C refrigerator and thawed overnight. After thawing, the culture medium was subpackaged into sterile 5 mL centrifuge tubes, with 3 mL per tube. Varying concentrations of mPEG-MAL-20KD-IL-12 prepared in Example 4 (2 ug/ml, 200 ng/ml, and 20 ng/ml), and mPEG-pALD-20KD-IL-12 prepared in Example 1 (800 ng/ml, 80 ng/ml, and 8 ng/ml) or different concentrations of IL-12 (100 ng/ml, 10 ng/ml, and 1 ng/ml) were added to the respective tubes. A portion of the medium was retained without any additions to serve as a control group. After the subpackage process, the tubes were stored at -20°C to prevent repeated freeze-thaw cycles. Prior to utilization, the tubes were thawed at 4°C or ambient temperature.

(4) The CD34+ cells were washed once with PBS by centrifugation at 300 g $\times$ 5 min. The supernatant was discarded, and the cell concentration was adjusted to 10 times the final inoculation concentration using Stemline II medium. For example, to achieve a final inoculation concentration of 1,000 cells per 1 mL of medium, the cell concentration needed to be adjusted to 10,000 cells/mL. Based on the results of preliminary experiments, the final inoculation concentration used in this study was 500 cells/mL. Therefore, the cell concentration should be adjusted to 5000 cells/mL.

(5) 300 ul of the cells with the pre-adjusted concentration was added to a 5 mL centrifuge tube containing 3 mL of hematopoietic stem cell colony medium (cell volume:colony medium volume = 1 : 10). The mixture was immediately and vigorously shaken to ensure thorough mixing of the cells with the medium. The mixture was allowed to stand at room temperature for 5-10 min to fully dissipate bubbles.

(6) A sterile 16-gauge syringe needle was securely fitted onto a syringe, and 1.1 mL of the cell-containing medium was slowly drawn up into a 35 mm cell culture dish. The 3.3 mL of mixture of the cells and cell medium contained in each 5 mL centrifuge tube was sufficient for inoculation into three 35 mm cell culture dishes, and the 3 culture dishes constitute 3 parallel samples.

(7) The culture dishes were slowly tilted and rotated so that the viscous culture medium evenly covered the entire culture dishes.

(8) 2 culture dishes inoculated with cells were placed in one 100 mm large culture dish, and one uncovered 35 mm culture dish was placed in the 100 mm large culture dish, and 3 mL of sterile water was added to the uncovered 35 mm culture dish, then the 100 mm large culture dish was covered with a lid, and placed in an incubator at 37°C, 5% $CO_2$, and humidity > 95% for 12 days. During the culture period, the water level in the uncovered 35 mm culture

dish was carefully monitored, and water was replenished as necessary.

(9) After 12 days of culture, an inverted microscope was used to observe the colonies, the type and number of colonies were classified and recorded, and the variations in number and types of colonies produced in different media were compared.

Fig. 13 shows the counts of CD34+ cells cultured under the stimulation of various concentrations of mPEG-MAL-20KD-IL-12 prepared in Example 4 (2 ug/ml, 200 ng/ml, and 20 ng/ml), mPEG-pALD-20KD-IL-12 prepared in Example 1 (800 ng/ml, 80 ng/ml, and 8 ng/ml), or various concentrations of IL-12 (100 ng/ml, 10 ng/ml, and 1 ng/ml). It can be found that compared with the group with polyethylene glycol-modified IL-12 or IL-12 stimulation, the number of cells in the blank control group decreased obviously on day 12, indicating that polyethylene glycol-modified IL-12 or IL-12 could maintain the survival of CD34+ cells for a longer time and slow down the apoptosis. For the higher concentration of IL-12, the stimulation effect on cells was not good, and appropriate concentrations, such as mPEG-MAL-20KD-IL-12 (20 ng/mL) and mPEG-pALD-20KD-IL-12 (80 ng/mL), could maintain the survival of the cells for a longer time.

Fig. 14 shows large red blood cell colonies (BFU-E) in the colony stimulation test.

Fig. 15 shows a single red blood cell colony (CFU-E) in the colony stimulation test.

Fig. 16 shows granulocyte/macrophage colonies (CFU-GM) in the colony stimulation test.

Fig. 17 shows a mixed colony of granulocyte/red blood cell/macrophage/megakaryocyte (CFU-GEMM) in the colony stimulation test.

Fig. 18 is a summary of the data from the colony stimulation test. Except for the mPEG-MAL-20K-IL-12 (2000 ng/mL) group, which had poorer effects than the blank control group, under the stimulation of other polyethylene glycol-modified IL-12 or IL-12, the differentiation direction of CD34+ was obviously different from that of the blank control, resulting in more CFU-GM or CFU-GEMM-type cell colonies. There was little difference in the number of other types of colonies, among which the mPEG-pALD -20K-IL-12 group and IL-12 group had better stimulation effect than the mPEG-MAL-20K-IL-12 group and the blank group, and the addition amount of mPEG-pALD-20K-IL-12 or IL-12 also had certain influence on the formation of cell colonies. Appropriate concentration (mPEG-pALD -20K-IL-12 (80 ng/mL) or IL-12 (10 ng/mL)) was beneficial to produce more CFU-GM colonies or CFU-GEMM colonies, while not affecting the formation of other colonies. High concentration of IL-12 (100 ng/mL) would affect the formation of BFU-E and CFU-E colonies. This may occur due to overstimulation, which caused a significant shift in the differentiation of CD34+ cells towards the granulocytes/macrophages. As a result, the proliferative and differentiation potential of the stem cells may be depleted, leading to an insufficient number of cells differentiating into the erythroid pathway.

## Example 16

This example provided an IL-12 stimulation experiment on cynomolgus monkeys after irradiation with radioactive radiation to evaluate the effect of polyethylene glycol derivative modified interleukin-12 on hemogram recovery. The specific experimental steps are as follows:

In this experiment, cynomolgus monkeys were randomly divided into three groups and irradiated with 500 Rad radiation simultaneously. In order to distribute the dose evenly, the first half of the dose was irradiated from front to back and the second half of the dose was irradiated from back to front. 24 h before receiving radiation or 30 min after receiving radiation, the experimental group was injected with mPEG-MAL-20K-IL-12 (2 ug/Kg) and IL-12 (100 ng/Kg) respectively, and the control group was injected with PBS buffer. Clinical signs were recorded twice daily. Decreased appetite (based on food intake) and physical activity were recorded daily and scored as follows: 1 = mild; 2 = moderate; and 3 = severe. Detailed physical examinations were performed before administration and twice weekly after administration. Body temperature (ear) was obtained before irradiation and on days 3, 10, 12, 14, 16, 18, 30, 45 and 60. Blood sampling (0.5 mL) for peripheral blood counting was performed before irradiation and on days 5, 10, 12, 14, 16, 18, 30, 45, and 60 to observe the dynamic changes of red blood cells, white blood cells, and platelets. In the early stage, the decline in the counts of various types of peripheral blood cells in the experimental group was less than that observed in the control group. The early recovery of platelets, red blood cells, and monocytes was significantly improved, and lymphocytes and neutrophils also recovered well. In the later stage, the experimental group that received injections of mPEG-MAL-20KD-IL-12 prepared in Example 4 exhibited a faster recovery rate of platelets, red blood cells, and monocytes compared to the experimental group that received injections of IL-12. This may be because PEGylated IL-12 had a longer half-life *in vivo,* so it could exert its effects over an extended period. Experimental results proved that IL-12 was useful in the prevention and treatment of hemogram abnormalities caused by myeloablative therapy, radiotherapy, chemotherapy, and acute radiation syndrome. mPEG-MAL-20KD-IL-12 had a longer half-life *in vivo* and a longer therapeutic effect due to the modification of IL-12 by PEG.

**Example 17**

This example provided an evaluation of the anti-tumor effect of polyethylene glycol derivative modified interleukin-12 via stimulating T cells to kill tumor cells by the polyethylene glycol derivative modified interleukin-12. The specific experimental steps are as follows:

(1) PBMC cells were obtained and placed in a culture plate coated with CD3 antibodies, RPMI 1640 medium was added to culture the cells, and T cells were harvested;
(2) Then the cells were inoculated at 2 × $10^6$/well into a 24-well plate, and different concentrations of mPEG-MAL-20K-IL-12 (2 ug/ml, 200 ng/ml, and 20 ng/ml) were added, while IL-12 (10 ng/mL, 1 pg/mL, and 0.01 pg/mL) was set as a control, and the same volume of buffer was added as a blank control, and the cells were continuously cultured for 7 days.
(3) At the same time, various cancer cells were cultured with RPMI 1640 medium. Cancer cells were labeled with 5 μmol/L Calcein AM in a 37°C water bath for 30 min. Then the medium was changed twice to remove the residual Calcein AM.
(4) Then PBMC was collected, inoculated into a 96-well plate at a ratio of 1/10 (i.e., 1 × $10^5$ target cells and 1 × $10^6$ effector cells), and incubated in a 37°C 5% $CO_2$ incubator for 24 h. At the same time, the target cells cultured alone was set as a blank control, and the lysis with triton lysis solution was set as the maximum killing well. Centrifugation was performed at 3000 r/min for 5 min and the supernatant was transferred to a new well.
(5) Fluorescence detection: A fluorescence measuring instrument was used to detect the fluorescence value (FI) of the supernatant. The excitation light was 485 nm and the emission light was 538 nm. The differences in the killing effect of PBMC on tumor cells under the stimulation of various concentrations of PEGylated interleukin-12 and IL-12 were evaluated based on the detection values.

**Example 18**

This example used a mouse tumor model to evaluate the *in vivo* anti-tumor effect of mPEG-MAL-20KD-IL-12 prepared in Example 4. Human hematopoietic stem cells (HSC) were transplanted into irradiated myeloablative M-NSG mice to construct CD34+ humanized mice, and pp65-MCF-7 cancer cells were transplanted into mice to create cancer models for evaluation of the *in vivo* anti-tumor activity of PEG-modified IL-12. 10 mice/group were given the specified dose of the test article every week (IP), and the experimental groups were injected with mPEG-MAL-20KD-IL-12 prepared in Example 4 (2 ug/Kg) and IL-12 (100 ng/Kg) respectively, and the control group was injected with PBS buffer. Tumor volume was measured before injection and on days 5, 10, 12, 14, 16, 18, and 30 after injection, and blood was collected to analyze lymphocyte activation/proliferation and blood IFN-y content. The differences in anti-tumor ability and side effects of mPEG-MAL-20K-IL-12 and IL-12 *in vivo* were statistically analyzed.

**Example 19**

This example provided long-term stimulation of NK-92 cells by mPEG-pALD-20KD-IL-12 (Example 1), mPEG-MAL-20KD-IL-12 (Example 4), and IL-12, and the long-term effect *in vitro* of polyethylene glycol-modified interleukin-12 was evaluated by cell counting and detection of IFN-γ release amount.

In the experiment, NK92 cells were divided into four groups, with two T25 bottles in each group, and 6 mL of NK92 cells was added into each bottle. The cell density was 1 × 10^5/mL, and 100 pg/mL of IL-12 or mPEG-MAL-20KD-IL-12 or mPEG-pALD-20KD-IL-12 was added to each group, respectively, or no drug was added as the control group. Then, the cells were subjected to stationary culture in a 5% $CO_2$ incubator at 37°C, and 120 μL was sampled on days 1, 2, 3, 5, 7, 9, 11, 13, and 15 respectively to detect the release amount of IFN-y, and 20 μL was sampled on days 3, 7, 11, and 15 respectively to detect the changes of number and viability of the cells.

The results are as shown below. The bar graph in Fig. 19 represents the number of cells, the number above the bar represents the viability of cells, and the dotted line graph represents the release amount of IFN-y. The results show that: 100 pg/mL IL-12 or mPEG-MAL-20KD-IL-12 or mPEG-pALD-20KD-IL-12 could stimulate NK92 cells to release excess IFN-y, and excess IFN-γ affected the proliferation of NK92 cells, resulting in the proliferation number and viability of NK92 cells significantly lower than that in the control group without adding drugs. The content of IFN-γ in the control group without drugs had obvious correlation with the number of cells. In the later stage of the experiment, the IFN-y content in the control group gradually decreased due to the decrease in the number of viable NK92 cells, but the IFN-y content in the group with adding drug remained at a high level. Especially for mPEG- MAL-20KD-IL-12 group and mPEG-pALD-20KD-IL-12 group, although the number of viable cells was not much different from that in the IL-12 group, the viability of the cells decreased more slowly, indicating that polyethylene glycol-modified IL-12 exhibited the ability to stimulate NK92 proliferation in the later stages of the experiment, can provide a longer-lasting stimulating effect than IL-12 and maintain the proliferation of NK92 cells, which was conducive to continuous treatment and better therapeutic effects.

**Example 20**

IL-12 could stimulate T cells to differentiate towards Th1, and could stimulate T cells or NK cells to produce pro-inflammatory cytokines, thereby improving the body's immunity and enhancing the body's resistance ability to viral infection, bacterial infection, parasitic infection and tumors. This example provided an evaluation of the effect on improving immunity, resistance to infection and anti-tumor of polyethylene glycol derivative modified interleukin-12 via stimulating NK cells to kill tumor cells by the polyethylene glycol derivative modified interleukin-12. The specific experimental steps are as follows:

(1) PBMC cells were obtained and NK cells were obtained through induction culture;
(2) Then the cells were inoculated at $1 \times 10^6$/well into a 24-well plate, and different concentrations of mPEG-MAL-20K-IL-12 (1 ug/ml, 10 ng/ml, and 100 pg/ml) and mPEG-pALD-20K-IL-12 (400 ng/mL, 4 ng/mL, and 40 pg/mL) were added, while IL-12 (50 ng/mL, 500 pg/mL, and 5 pg/mL) was set as a control, and the same volume of buffer was added as a blank control, and the cells were continuously cultured for 7 days.
(3) At the same time, four types of cancer cells, Jurkat, K562, SK-BR3, and NCI-N87, were cultured with RPMI 1640 medium. The cancer cells were labeled with 5 μmol/L Calcein AM in a 37°C water bath for 30 min. Then the medium was changed twice to remove the residual Calcein AM.
(4) NK cells were then collected, inoculated into a 96-well plate according to different effector cell: target cell ratios (NK : Jurkat = 1 : 3, NK : K562 = 1 : 1, NK : SK-BR3 = 2 : 1, NK : NCI-N87 = 2 : 1), and incubated for 24 h in a 5% $CO_2$ incubator at 37°C. Centrifugation was performed at 1300 r/min for 5 min, the cells were incubated and stained with PI fluorescent dye at 37°C for 15 min and then the cells were centrifuged and resuspended with PBS.
(5) Analysis with flow cytometer.

Figs. 20 to 23 are charts of flow cytometry results of NK killing Jurkat, K562, SK-BR3, and NCI-N87 respectively. The cell population in the upper right corner that is Calcein-AM+PI+ double positive represents the population of killed tumor cells. The cell population in the lower right corner that is Calcein-AM+PI- represents the population of live tumor cells. The cell population in the lower left corner that is Calcein-AM-PI- represents the population of live NK cells. The cell population in the upper left corner that is Calcein-AM-PI+ represents the population of dead NK cells. It can be seen from the figures that NK cells stimulated by different concentrations of IL-12, mPEG-MAL-20K-IL-12 and mPEG-pALD-20K-IL-12 have different killing effects on tumor cells, with the high concentration being the most effective and the low concentration the least effective. In the same tumor, there is little difference in the killing effect of mPEG-MAL-20K-IL-12 and mPEG-pALD-20K-IL-12 at the three concentrations of high, medium and low. Particularly, the low concentration has a better effect compared with IL-12. This is mainly because the PEGylation modification enhances the stability of IL-12, so that IL-12 would not be easily degraded by enzyme or endocytosis, and could continue to function and stimulate tumor killing.

Fig. 24 is a summary of the flow cytometric detection results of NK killing SK-BR3. It can be seen from the figure that polyethylene glycol-modified IL-12 has better killing effect than natural interleukin-12 at three concentrations: high, medium and low. Particularly, the high concentration group of mPEG-MAL-20K-IL-12 (1 ug/mL) and the low concentration group of mPEG-pALD-20K-IL-12 (40 pg/mL) have more significant effects compared with the corresponding groups of natural IL-12.

Fig. 25 is a summary of the flow cytometric detection results of NK killing NCI-N87. It can be seen from the figure that polyethylene glycol-modified IL-12 has better killing effect than natural interleukin-12 at three concentrations: high, medium and low. Particularly, both the high concentration group and the medium concentration group have more significant killing effects, and the medium concentration group of mPEG-MAL-20K-IL-12 (10 ng/mL), the medium concentration group of mPEG-pALD-20K-IL-12 (4 ng/mL) and the low concentration group of mPEG-pALD-20K-IL-12 (40 pg/mL) have more significant effects compared with the corresponding groups of natural IL-12.

**Example 21**

The stability of the sample was evaluated by analyzing the particle size distribution of protein from multiple perspectives, that is, detecting natural IL-12(S1), mPEG-MAL-20K-IL-12 (S2) and mPEG-pALD-20K-IL-12 (S3) using high-throughput protein stability analysis system (Uncle), and analyzing the result parameters Tm (protein melting temperature), Tagg (protein aggregation temperature), Z-Ave.Dia (average protein particle size), PDI (particle size polydispersity), Pk1. Mode Dia, Pk1 Mass% and other parameters.

Table 9 is a summary of the results. It can be seen that polyethylene glycol modification could increase the Tm

value and Tagg value of IL-12, and could also increase the particle size of the protein while maintaining a relatively uniform dispersity.

Table 9:

| Sample | Tm | | | Tagg 266 | | | Size | | | |
|---|---|---|---|---|---|---|---|---|---|---|
| | Tm (°C) | Average (°C) | % CV | Tagg 266 (°C) | Average (°C) | % CV | Z-Ave.Dia | PDI | Pk 1 Mode Dia. (nm) | Pk 1 Mass (%) |
| S1 | 60.30 | 60.30 | 0.00 | 59.32 | 59.08 | 0.57 | 7.80 | 0.067 | 8.23 | 100 |
| S1 | 60.30 | | | 58.84 | | | 7.78 | 0.046 | 8.29 | 100 |
| S2 | 61.17 | 61.18 | 0.01 | 60.70 | 60.55 | 0.35 | 11.41 | 0.054 | 11.30 | 100 |
| S2 | 61.18 | | | 60.40 | | | 11.56 | 0.028 | 11.25 | 100 |
| S3 | 61.40 | 61.35 | 0.12 | 60.20 | 60.18 | 0.06 | 11.83 | 0.049 | 12.17 | 100 |
| S3 | 61.30 | | | 60.15 | | | 11.84 | 0.046 | 12.18 | 100 |

Fig. 26 shows a comparative chart of denaturation curves and aggregation curves of natural IL-12(S1), mPEG-MAL-20K-IL-12 (S2) and mPEG-pALD-20K-IL-12 (S3). The three do not show significant differences in their denaturation curves, but the polyethylene glycol-modified interleukin-12 has better performance in aggregation curve. With the increase of temperature, natural IL-12 forms an increasing number of aggregates. Although mPEG-MAL-20K-IL-12 and mPEG-pALD-20K-IL-12 also form aggregates, respectively, the aggregates are less, and the amount of aggregates formed does not increase significantly with rising temperature. This indicates that the polyethylene glycol modification is beneficial to improve the stability of IL-12 and maintain the original heterodimer form of IL-12 instead of forming larger aggregates. Therefore, the excellent stability of polyethylene glycol-modified interleukin-12 has better advantages in later storage, transportation, preparation production and use.

## Claims

**1.** A polyethylene glycol derivative modified interleukin-12 obtained by performing a modification reaction on a polyethylene glycol derivative and interleukin-12, wherein an active group of the polyethylene glycol derivative and an active group of the interleukin-12 participate in the modification reaction; the active group of the polyethylene glycol derivative comprises one or more of an aldehyde group, a ketone group, a maleimide group, a vinyl sulfone group, a terminal alkyne group, a succinimide group, a p-nitrophenyl group, an isobutyl chloroformate group, a halo group, a thio group and a carboxyl group; and the active group of the interleukin-12 comprises one or more of an N-terminal $\alpha$-amino group, an $\varepsilon$-amino group of a lysine side chain and a free sulfhydryl group of a cysteine.

**2.** The polyethylene glycol derivative modified interleukin-12 of claim 1, wherein:

the active group of the polyethylene glycol derivative is an aldehyde group, a ketone group, a succinimide group, an isobutyl chloroformate group, a p-nitrophenyl group or a carboxyl group, and the active group of the interleukin-12 that undergoes the modification reaction is an N-terminal $\alpha$-amino group; or

the active group of the polyethylene glycol derivative is a maleimide group, a vinyl sulfone group, a terminal alkyne group, a halo group or a thio group, and the active group of the interleukin-12 that undergoes the modification reaction is a free sulfhydryl group of a cysteine, preferably a free sulfhydryl group of a cysteine at position C252 of the P40 subunit; or

the active group of the polyethylene glycol derivative is a succinimide group, an isobutyl chloroformate group, a p-nitrophenyl group or a carboxyl group, and the active group of the interleukin-12 that undergoes the modification reaction is an $\varepsilon$-amino group of a lysine side chain.

**3.** The polyethylene glycol derivative modified interleukin-12 of claim 1 or 2, wherein the molecular weight of the polyethylene glycol derivative is 5-40 KD, preferably 10-30 KD, and more preferably 20 KD.

**4.** The polyethylene glycol derivative modified interleukin-12 of any one of claims 1 to 3, wherein the polyethylene glycol derivative comprises one or more of a polyethylene glycol aldehyde derivative, a polyethylene glycol ketone

derivative, a polyethylene glycol active ester derivative, a polyethylene glycol carboxyl derivative, a polyethylene glycol unsaturated bond-containing derivative, a polyethylene glycol haloalkane derivative and a polyethylene glycol sulfur activity-containing derivative;
preferably, wherein:

the polyethylene glycol aldehyde derivative comprises one or more of a polyethylene glycol acetaldehyde derivative, a polyethylene glycol propionaldehyde derivative and a polyethylene glycol butyraldehyde derivative; more preferably, the polyethylene glycol propionaldehyde derivative comprises monomethoxy polyethylene glycol propionaldehyde and/or two-arm polyethylene glycol propionaldehyde;
the polyethylene glycol active ester derivative comprises one or more of a polyethylene glycol succinimide active ester derivative, a polyethylene glycol p-nitrobenzene active ester derivative and a polyethylene glycol isobutyl chloroformate active ester derivative; more preferably, the polyethylene glycol succinimide active ester derivative comprises monomethoxy polyethylene glycol succinimide succinate and/or monomethoxy polyethylene glycol succinimide carbonate;
the polyethylene glycol carboxyl derivative comprises one or more of a polyethylene glycol acetic acid derivative, a polyethylene glycol propionic acid derivative, a polyethylene glycol butyric acid derivative, a polyethylene glycol valeric acid derivative, a polyethylene glycol hexanoic acid derivative, a polyethylene glycol oxalic acid derivative, a polyethylene glycol malonic acid derivative, a polyethylene glycol succinic acid derivative, a polyethylene glycol glutaric acid derivative and a polyethylene glycol hexanedioic acid derivative; more preferably, the polyethylene glycol carboxyl derivative comprises monomethoxy polyethylene glycol acetic acid;
the polyethylene glycol unsaturated bond-containing derivative comprises one or more of polyethylene glycol vinyl sulfones, polyethylene glycol maleimides, and polyethylene glycol terminal acetylenes; more preferably, the polyethylene glycol unsaturated bond-containing derivative comprises one or more of monomethoxy polyethylene glycol vinyl sulfone, monomethoxy polyethylene glycol maleimide and two-arm polyethylene glycol maleimide;
the polyethylene glycol haloalkane derivative comprises one or more of a polyethylene glycol iodopropionamide derivative, a polyethylene glycol iodoacetamide derivative, a polyethylene glycol chloropropionamide derivative, a polyethylene glycol chloroacetamide derivative, a polyethylene glycol bromopropionamide derivative, a polyethylene glycol bromoacetamide derivative, a polyethylene glycol fluoropropionamide derivative and a polyethylene glycol fluoroacetamide derivative; more preferably, the polyethylene glycol haloalkane derivative comprises monomethoxy polyethylene glycol iodoacetamide;
the polyethylene glycol sulfur activity-containing derivative comprises one or more of polyethylene glycol dithioisopyridines, polyethylene glycol thiosulfonates, and polyethylene glycol thiols; more preferably, the polyethylene glycol sulfur activity-containing derivative comprises one or more of monomethoxy polyethylene glycol dithioisopyridine, monomethoxy polyethylene glycol thiomethanesulfonate and monomethoxy polyethylene glycol thiobenzenesulfonate.

**5.** The polyethylene glycol derivative modified interleukin-12 of claim 1, comprising one or more of the following: monomethoxy polyethylene glycol propionaldehyde-modified interleukin-12, two-arm polyethylene glycol propionaldehyde-modified interleukin-12, monomethoxy polyethylene glycol succinimide succinate-modified interleukin-12, monomethoxy polyethylene glycol maleimide-modified interleukin-12, branched monomethoxy polyethylene glycol maleimide-modified interleukin-12, monomethoxy polyethylene glycol succinimide carbonate-modified interleukin-12, monomethoxy polyethylene glycol succinimide carbonate-modified interleukin-12, monomethoxy polyethylene glycol vinyl sulfone-modified interleukin-12, monomethoxy polyethylene glycol iodoacetamide-modified interleukin-12, monomethoxy polyethylene glycol dithioisopyridine-modified interleukin-12, and monomethoxy polyethylene glycol acetic acid-modified interleukin-12.

**6.** A preparation method of the polyethylene glycol derivative modified interleukin-12 of any one of claims 1 to 5, comprising the following steps:

dissolving interleukin-12 in a solvent to prepare an interleukin-12 solution;
dissolving a polyethylene glycol derivative in water to prepare an aqueous solution of the polyethylene glycol derivative;
dropwise adding the aqueous solution of the polyethylene glycol derivative to the interleukin-12 solution for a reaction under stirring;
performing ultrafiltration to remove impurities after the reaction, and then performing separation and purification by chromatography to obtain the polyethylene glycol derivative modified interleukin-12.

**7.** The preparation method of claim 6, wherein :

the chromatography comprises ion exchange chromatography, size exclusion chromatography, hydrophobic chromatography or reversed phase chromatography;
the molar ratio of the polyethylene glycol derivative to the interleukin-12 is (5.5-20) : 1.

**8.** The preparation method of claim 6, wherein :

the polyethylene glycol derivative is a polyethylene glycol aldehyde derivative or a polyethylene glycol ketone derivative, and the solvent comprises an acidic buffer solution with a pH value of 4 to 6.5 so as to control a reaction condition to be an acidic environment; a reducing agent further needs to be added during the reaction; or
the polyethylene glycol derivative is a polyethylene glycol active ester derivative, and the solvent comprises an alkaline buffer solution with a pH value of 7.5 to 10 so as to control a reaction condition to be an alkaline environment; or
the polyethylene glycol derivative is a polyethylene glycol carboxyl derivative, a polyethylene glycol unsaturated bond-containing derivative, a polyethylene glycol haloalkane derivative or a polyethylene glycol sulfur activity-containing derivative, and the solvent comprises a neutral or near-neutral buffer solution with a pH value of 6 to 8 so as to control a reaction condition to be a neutral or near-neutral environment;
and the temperature of the reaction under stirring is 0-40°C, preferably 10°C.

**9.** Use of the polyethylene glycol derivative modified interleukin-12 of any one of claims 1 to 5 in the preparation of a medicament with one or more of the following efficacies:
improving immunity; resistance to infection; preventing and/or treating a disease with abnormal hemogram; and preventing and/or treating a tumor.

**10.** The use of claim 9, wherein:

the resistance to infection comprises resistance to one or more of viral infection diseases, bacterial infection diseases and parasitic infection diseases; the resistance to viral infection diseases comprises resistance to one or more of HSV, HIV, hepatitis B and hepatitis C; the resistance to bacterial infection diseases comprises resistance to one or more of pulmonary tuberculosis, salmonellosis and listeriosis; the resistance to parasitic infection diseases comprises resistance to one or more of malaria, leishmaniasis and schistosomiasis;
the disease with abnormal hemogram comprises: one or more of a disease with abnormal hemogram caused by a hematopoietic malignant tumor, a disease with abnormal hemogram caused by a radiotherapy, a chemotherapy, or a myeloablative therapy, and a disease with abnormal hemogram caused by acute radiation syndrome;
the tumor comprises various types of solid tumors and/or non-solid tumors.

**11.** A pharmaceutical preparation, comprising: an effective amount of the polyethylene glycol derivative modified interleukin-12 of any one of claims 1 to 5; and a pharmaceutically acceptable excipient.

**12.** The pharmaceutical preparation of claim 11, wherein the pharmaceutical preparation is a liquid preparation and/or a lyophilized preparation.

**13.** The pharmaceutical preparation of claim 11, wherein the administration route of the pharmaceutical preparation comprises injection, oral administration, external application or administration via a combination of multiple routes; preferably, a treatment method using the pharmaceutical preparation comprises one of or a combination of a radiotherapy, a chemotherapy, a myeloablative therapy, a CAR-T therapy, a CAR-NK therapy, an antibody-targeted therapy, an oncolytic virus therapy and a gene vaccine therapy.

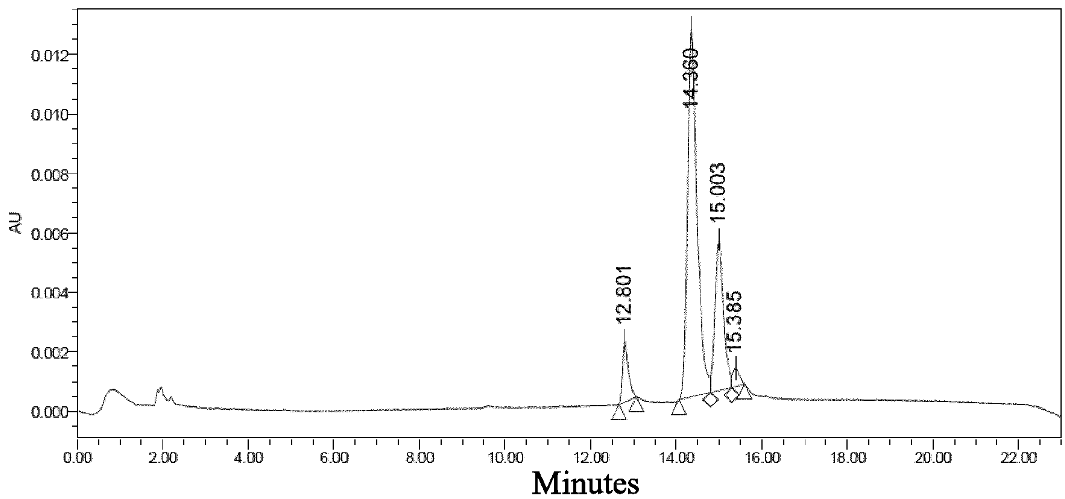


*FIG. 1*

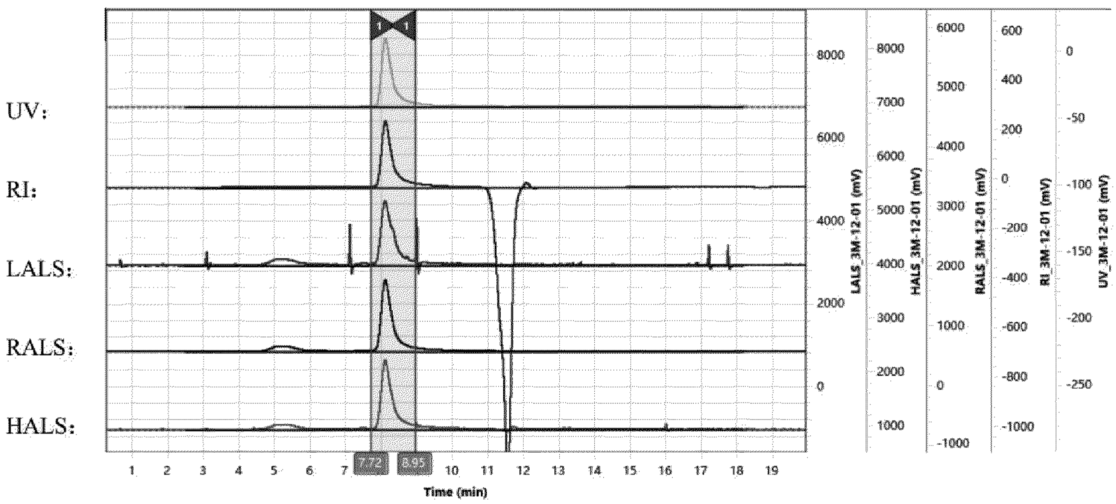


*FIG. 2*

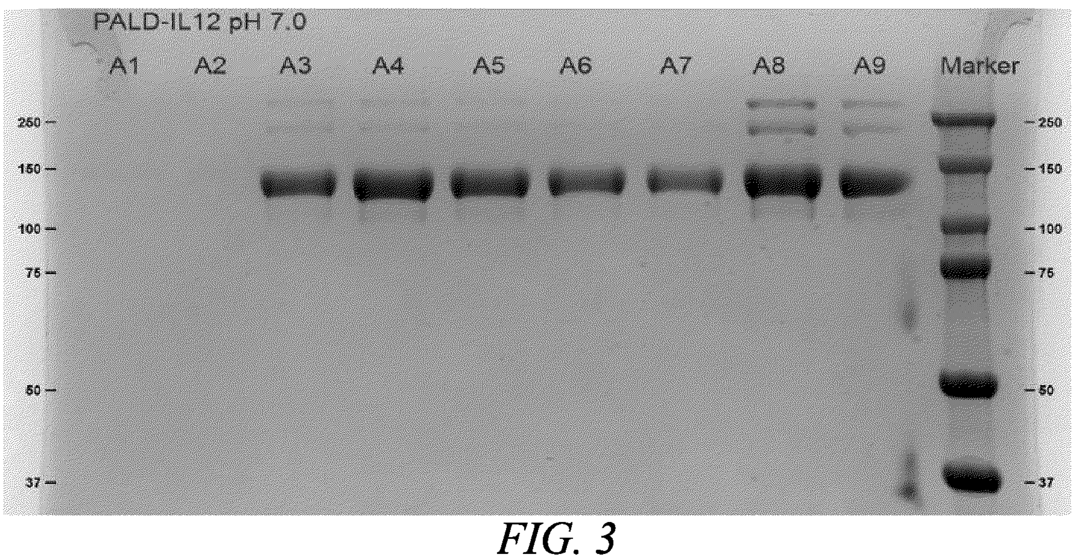


*FIG. 3*

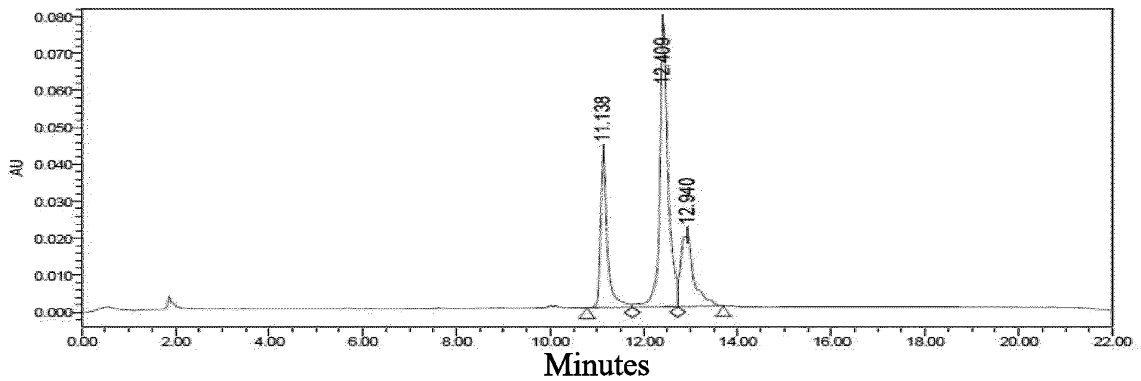


*FIG. 4*

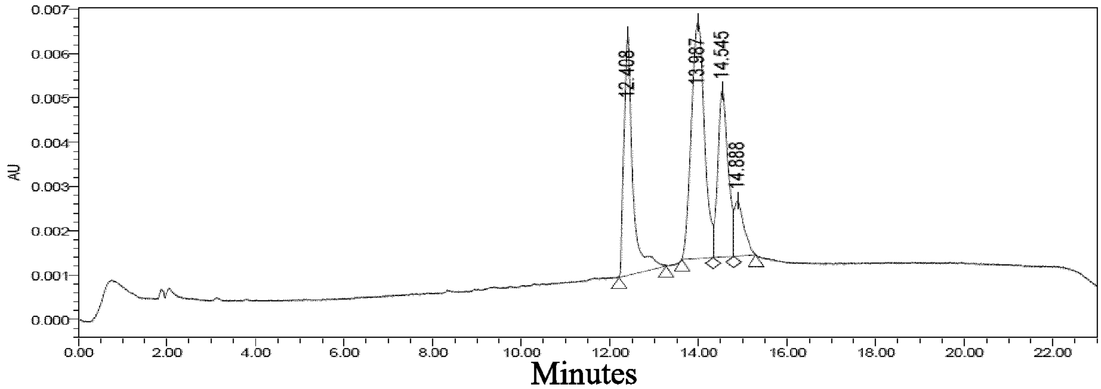


*FIG. 5*

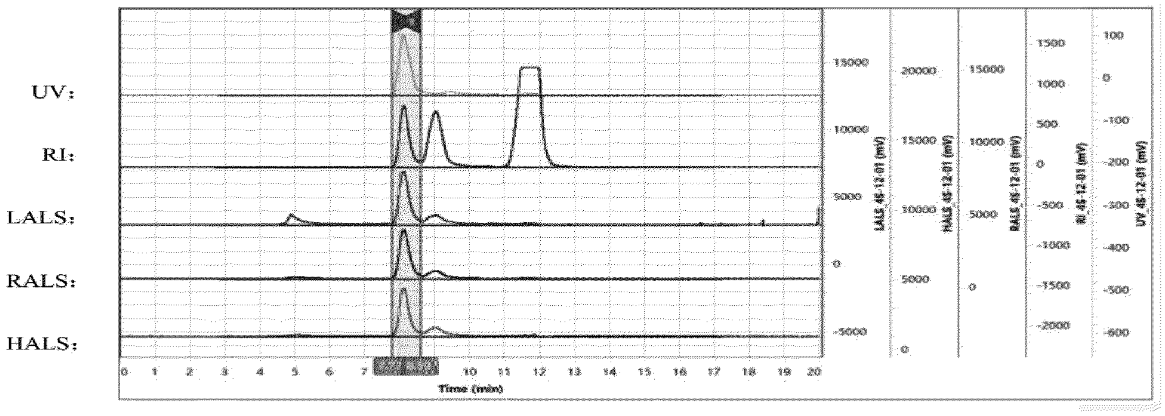


*FIG. 6*

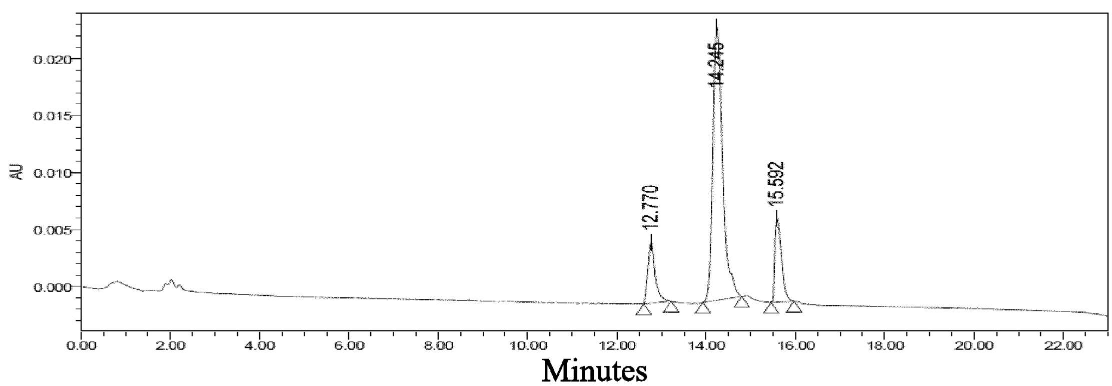


*FIG. 7*

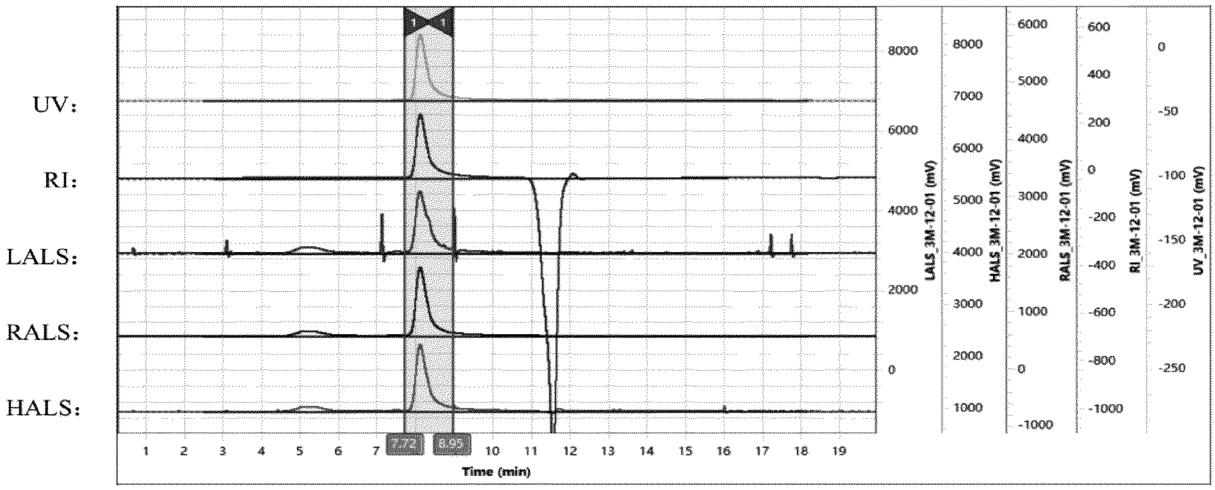
UV:
RI:
LALS:
RALS:
HALS:
7.72
8.95
Time (min)
LALS_3M-12-01 (mV)
HALS_3M-12-01 (mV)
RALS_3M-12-01 (mV)
RI_3M-12-01 (mV)
UV_3M-12-01 (mV)

*FIG. 8*

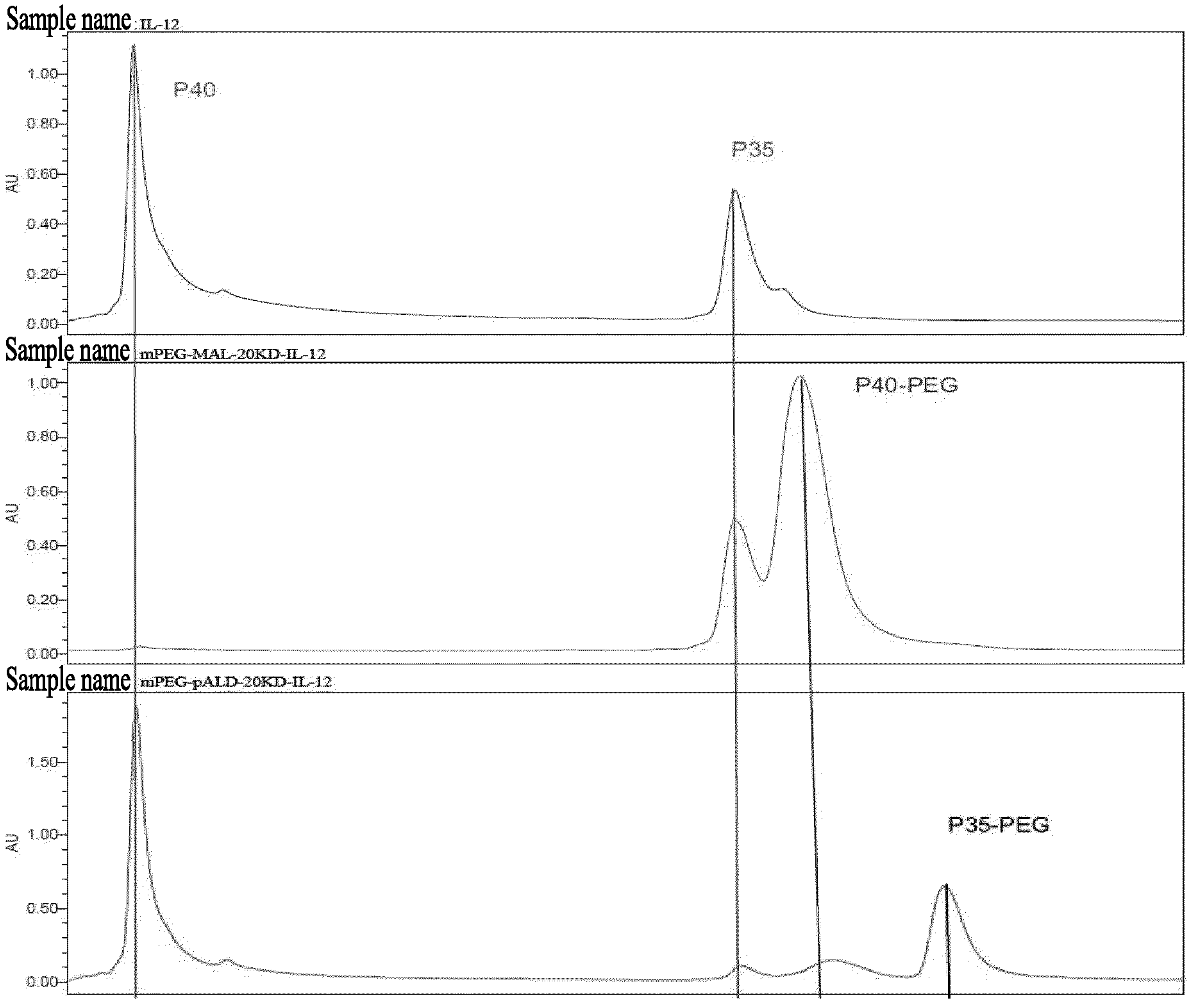
Sample name IL-12
P40
P35
Sample name mPEG-MAL-20KD-IL-12
P40-PEG
Sample name mPEG-pALD-20KD-IL-12
P35-PEG
AU

*FIG. 9*

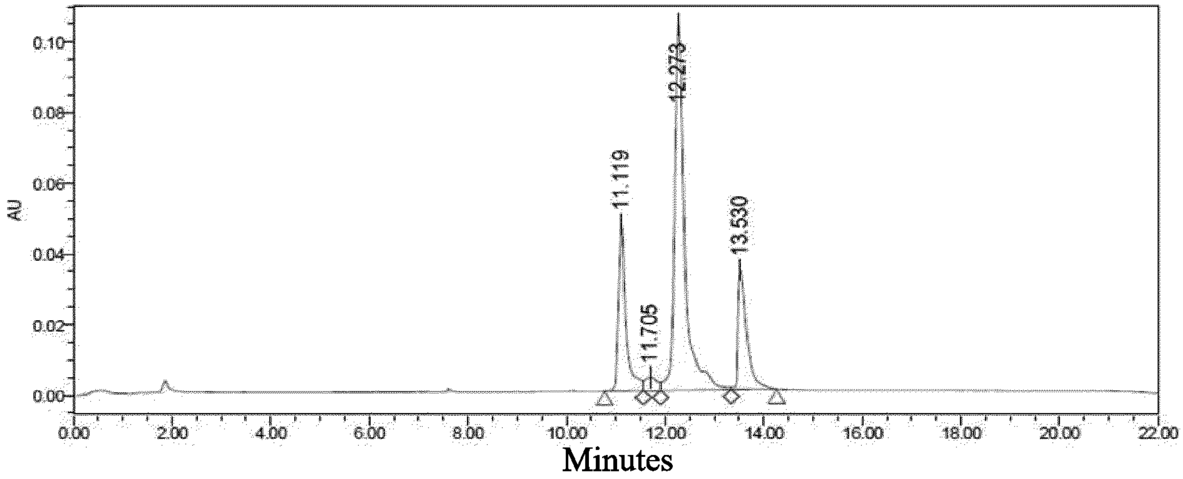


*FIG. 10*

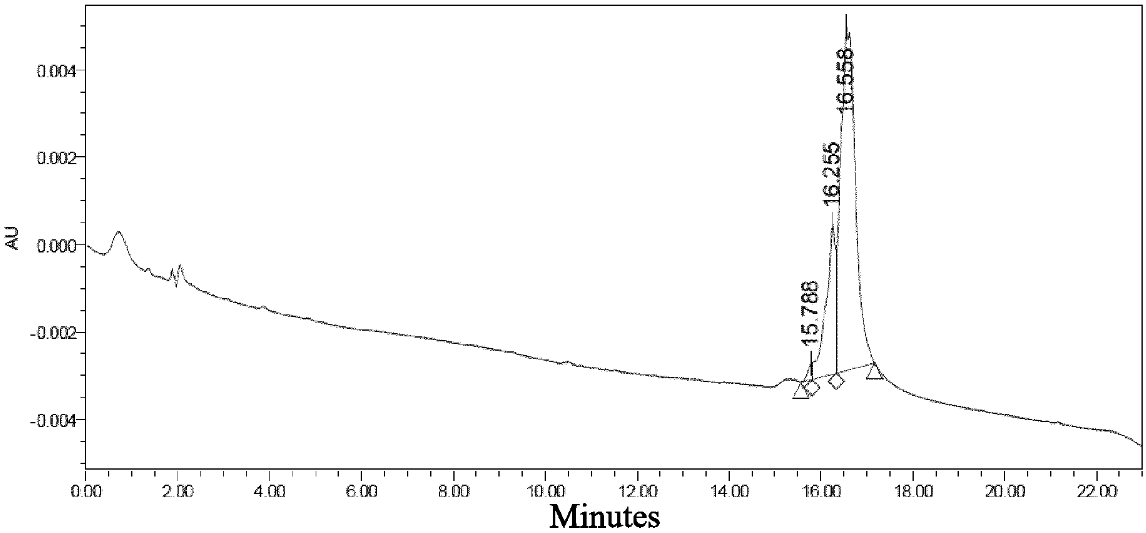


*FIG. 11*

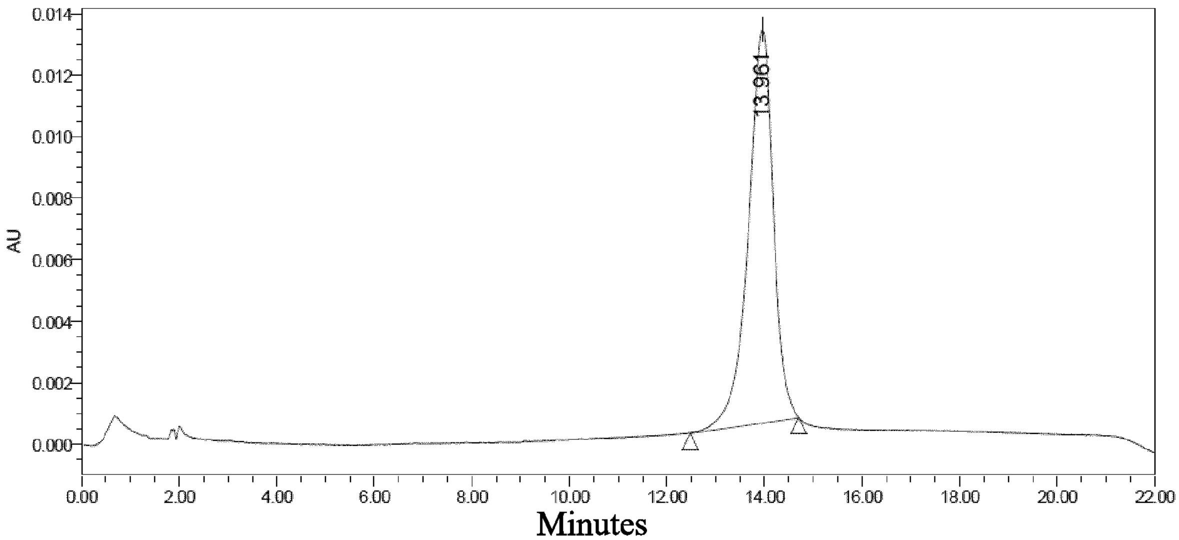


*FIG. 12*

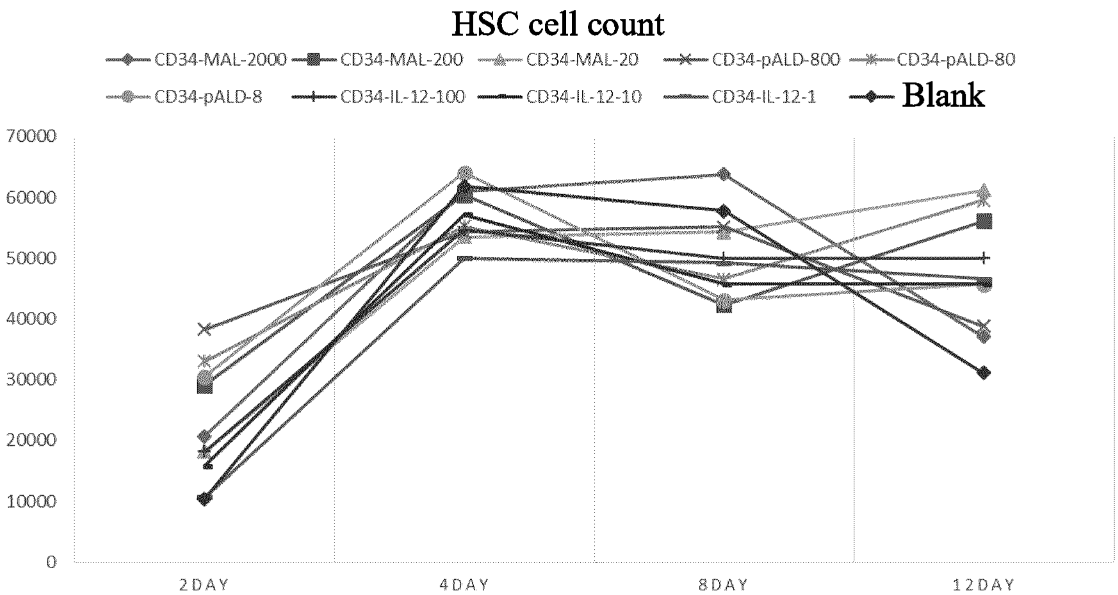


*FIG. 13*

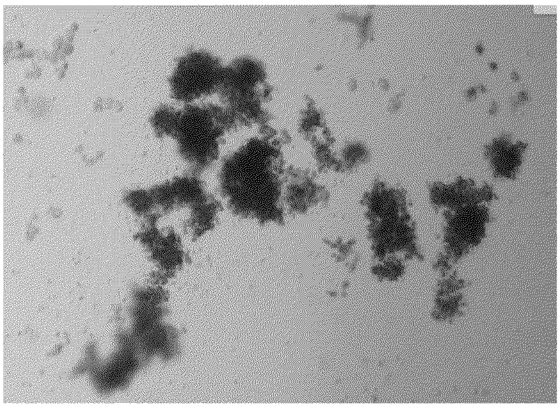

*FIG. 14*

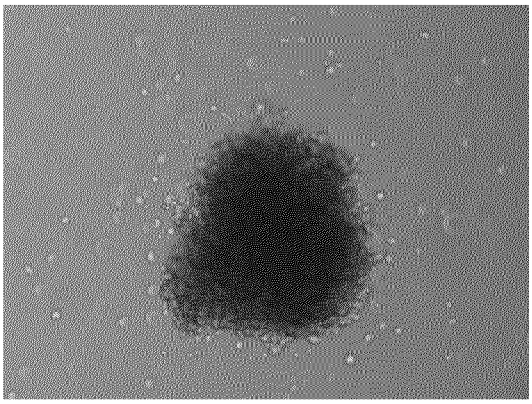

*FIG. 15*

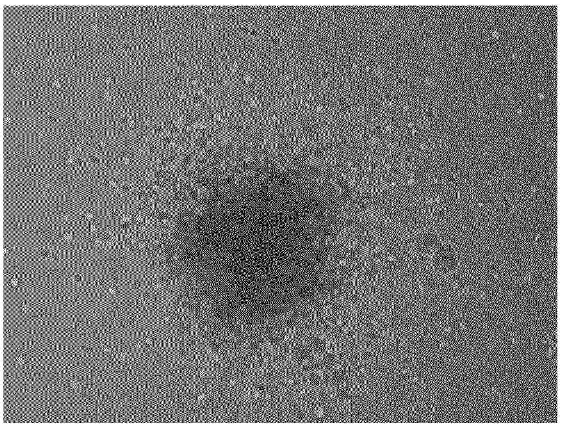

*FIG. 16*

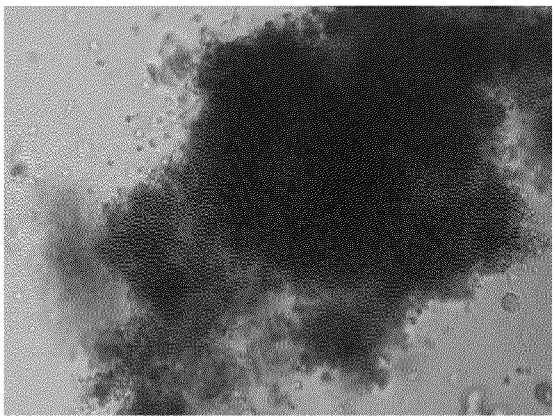

*FIG. 17*

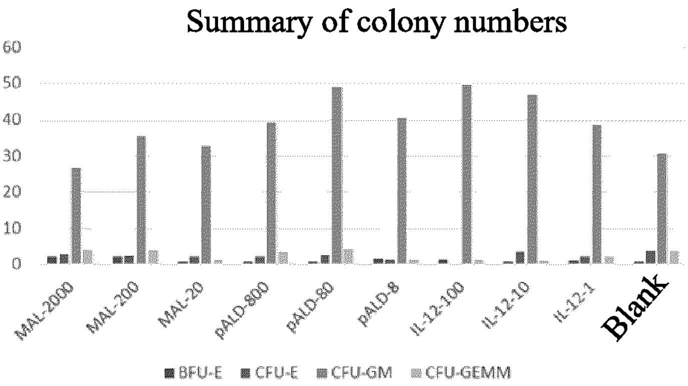


*FIG. 18*

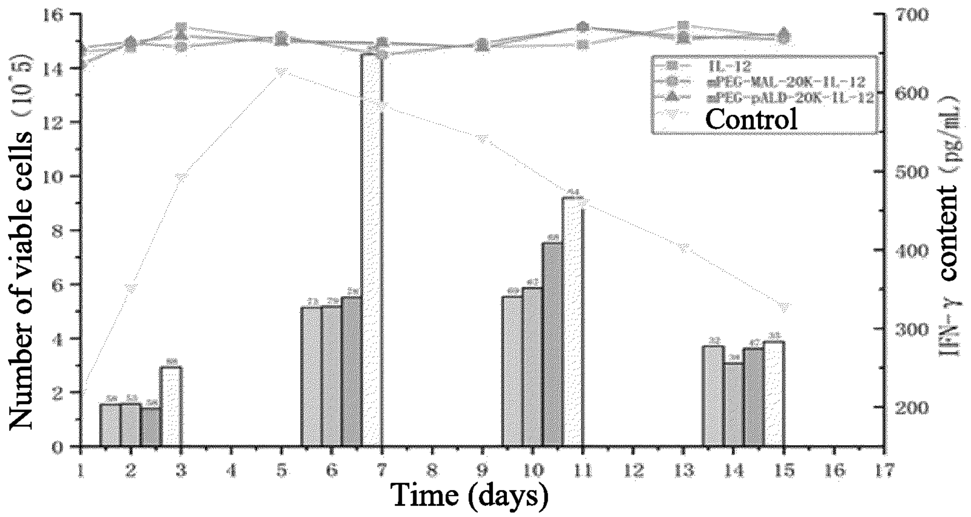


*FIG. 19*

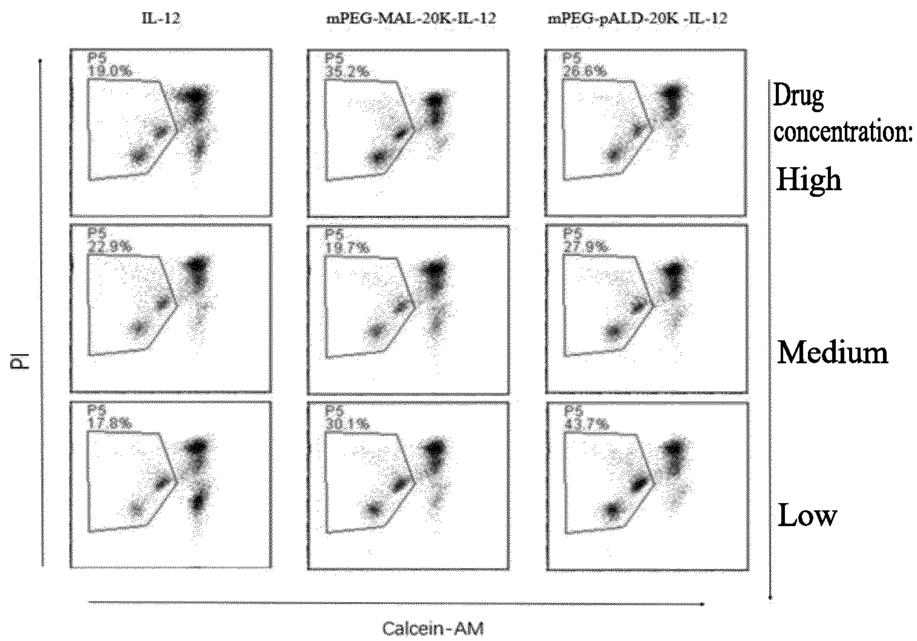


*FIG. 20*

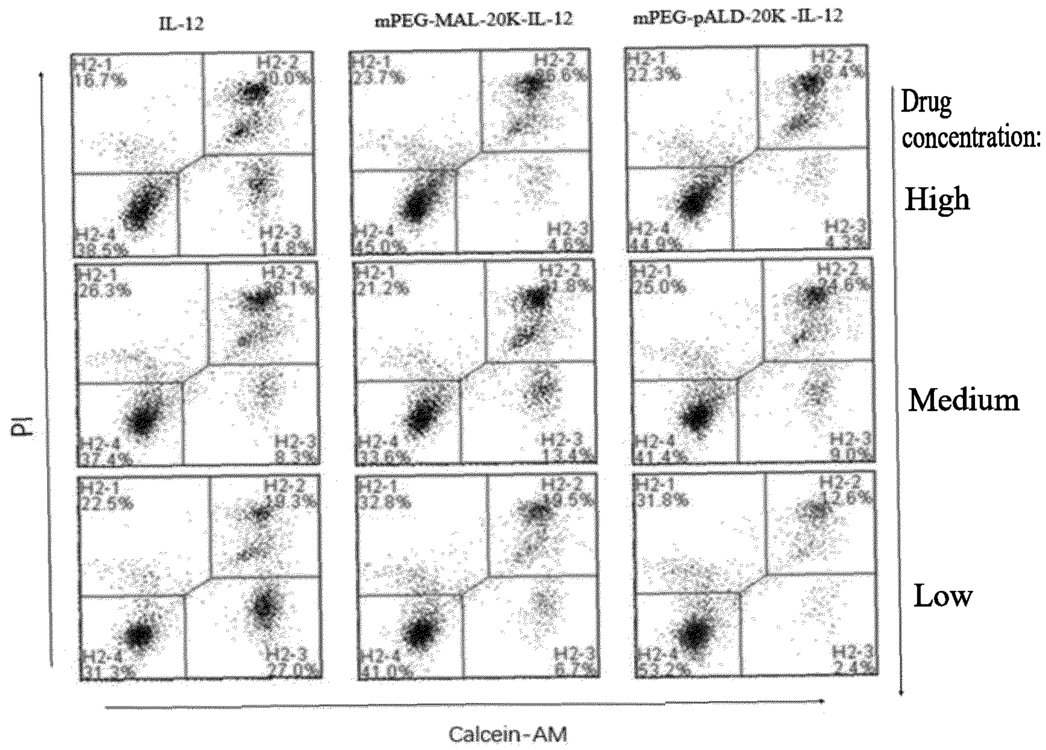
IL-12
mPEG-MAL-20K-IL-12
mPEG-pALD-20K -IL-12
Drug concentration:
High
Medium
Low
PI
Calcein-AM

*FIG. 21*

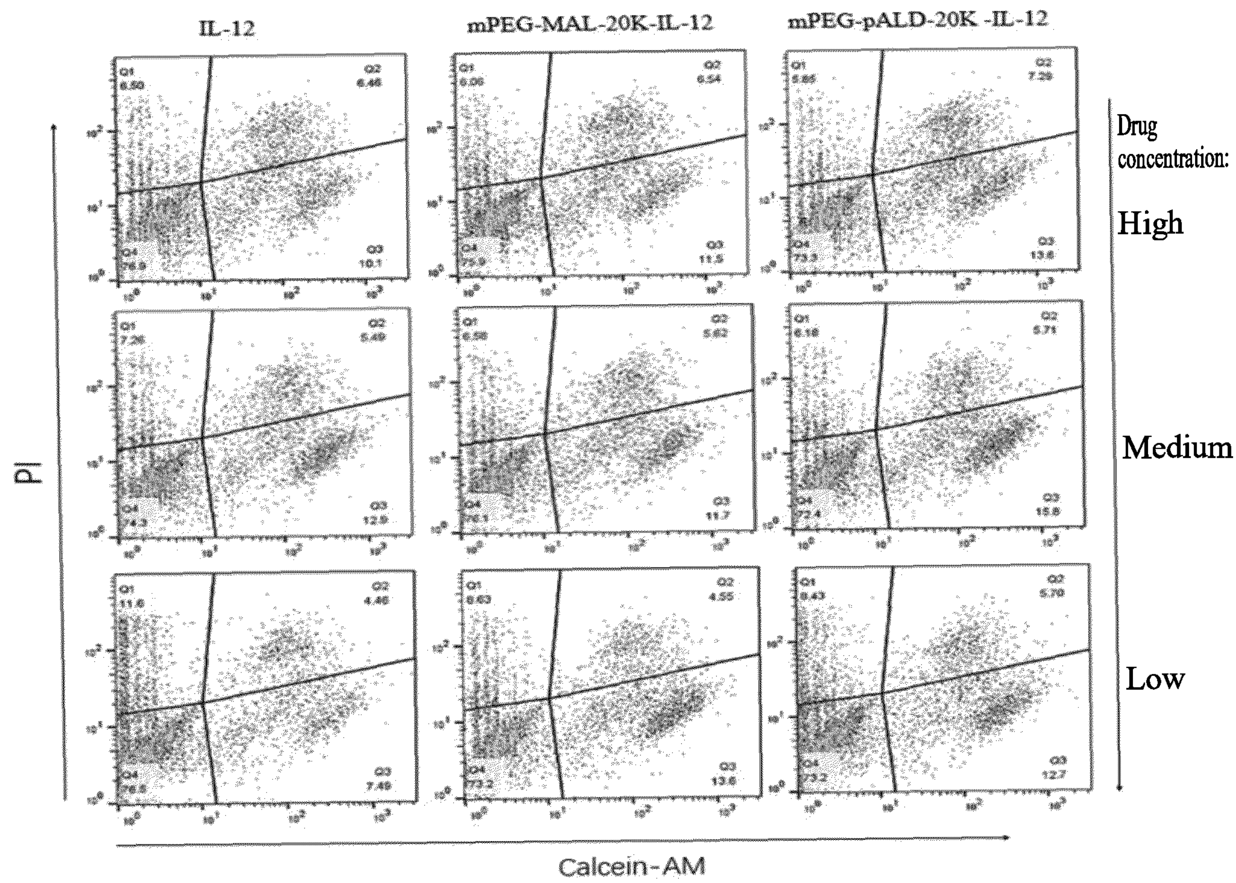
IL-12
mPEG-MAL-20K-IL-12
mPEG-pALD-20K -IL-12
Drug concentration:
High
Medium
Low
PI
Calcein-AM

*FIG. 22*

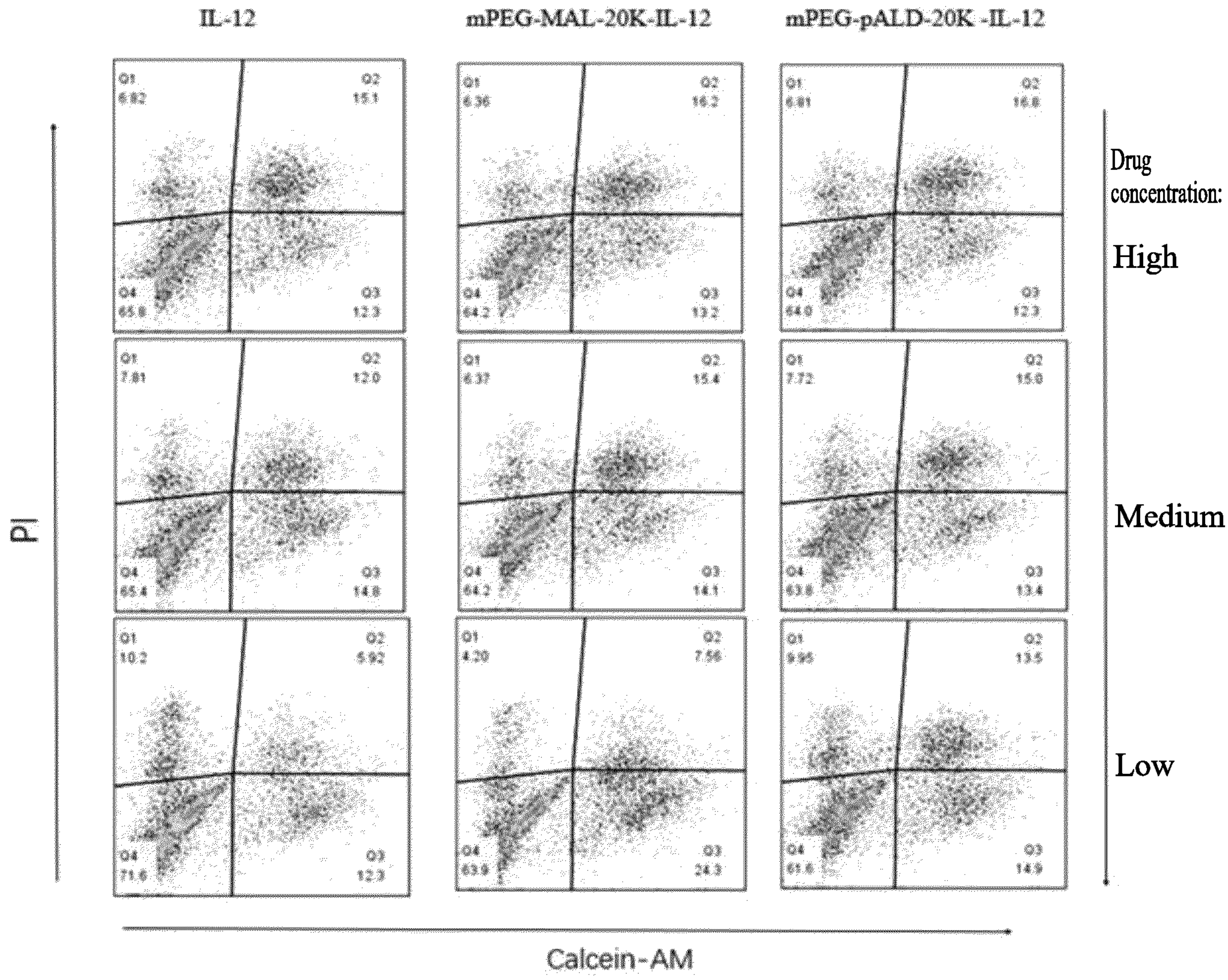
IL-12
mPEG-MAL-20K-IL-12
mPEG-pALD-20K -IL-12
Drug concentration:
High
Medium
Low
PI
Calcein-AM

FIG. 23

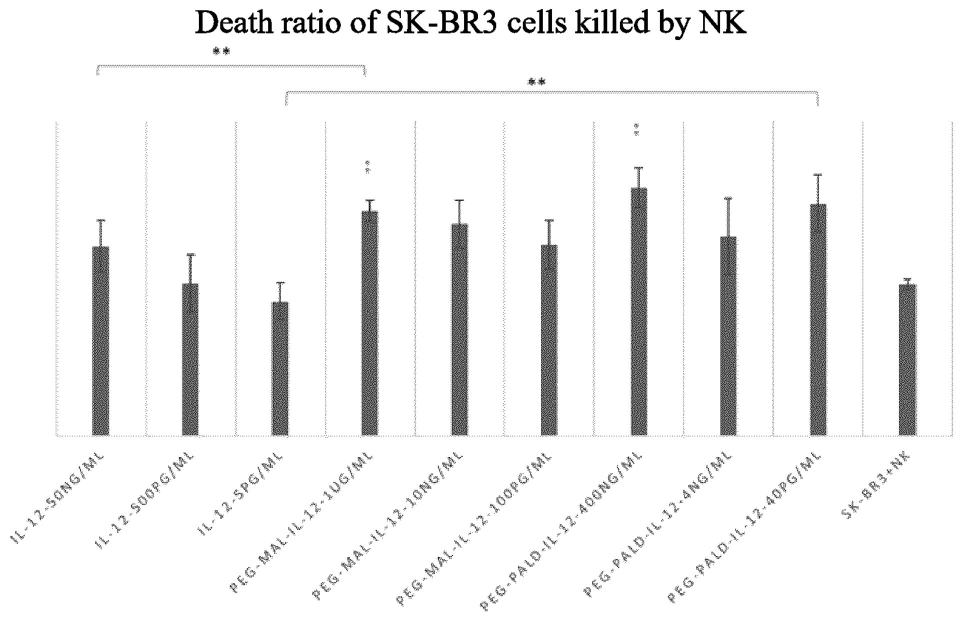
Death ratio of SK-BR3 cells killed by NK
**
**
IL-12-50NG/ML
IL-12-500PG/ML
IL-12-5PG/ML
PEG-MAL-IL-12-1UG/ML
PEG-MAL-IL-12-10NG/ML
PEG-MAL-IL-12-100PG/ML
PEG-PALD-IL-12-400NG/ML
PEG-PALD-IL-12-4NG/ML
PEG-PALD-IL-12-40PG/ML
SK-BR3+NK

FIG. 24

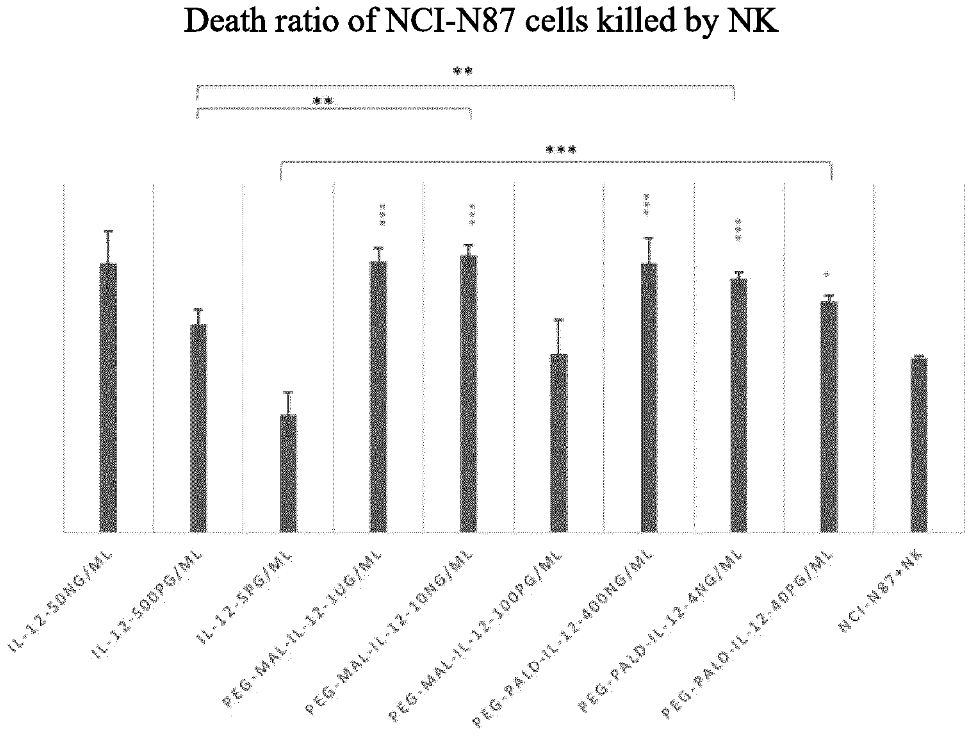
Death ratio of NCI-N87 cells killed by NK
**
**
***
***
***
***
***
*
IL-12-50NG/ML
IL-12-500PG/ML
IL-12-5PG/ML
PEG-MAL-IL-12-1UG/ML
PEG-MAL-IL-12-10NG/ML
PEG-MAL-IL-12-100PG/ML
PEG-PALD-IL-12-400NG/ML
PEG-PALD-IL-12-4NG/ML
PEG-PALD-IL-12-40PG/ML
NCI-N87+NK

*FIG. 25*

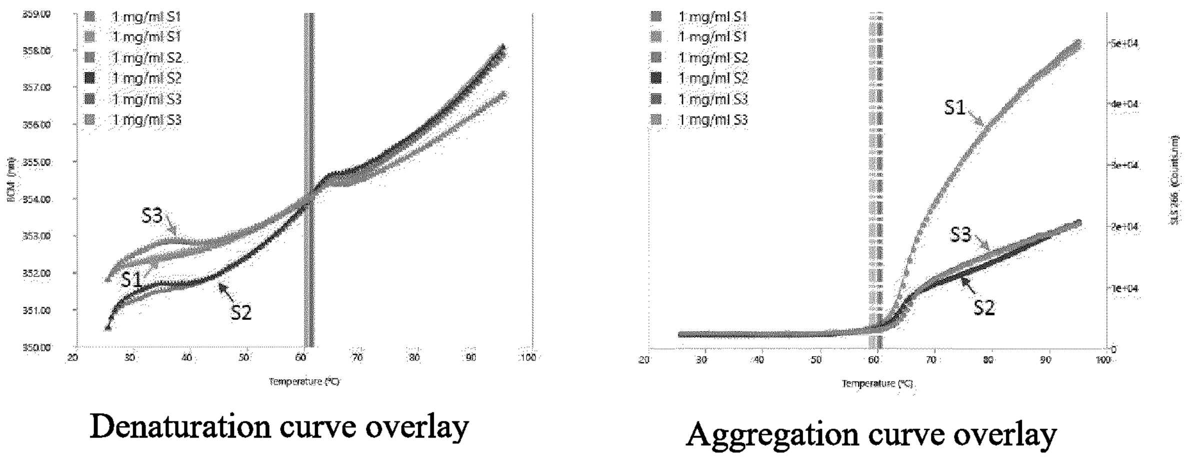
1 mg/ml S1
1 mg/ml S1
1 mg/ml S2
1 mg/ml S2
1 mg/ml S3
1 mg/ml S3
S1
S2
S3
Temperature (°C)
Denaturation curve overlay
Aggregation curve overlay

*FIG. 26*

## INTERNATIONAL SEARCH REPORT

International application No. **PCT/CN2022/123400**

**A. CLASSIFICATION OF SUBJECT MATTER**

C07K 14/54(2006.01)i; C07K 1/02(2006.01)i; A61P 35/00(2006.01)i; A61P 31/00(2006.01)i; A61K 38/20(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNABS; CNKI; CNTXT; DWPI; WPABS; ENTXT; OETXT; Web of Science: 聚乙二醇, 聚氧乙烯醇, 聚氧乙烯二醇, 单甲氧聚乙二醇, 白介素12, IL 12, IL-12, IL, 肿瘤, 癌, 细胞因子, 半衰期, PEP, polyethylene glycol, methoxy polyethylene glycol, Interleukin 12, Interleukin, tumor, cancer, cell factor, half life等.

**C. DOCUMENTS CONSIDERED TO BE RELEVANT**

| Category* | Citation of document, with indication, where appropriate, of the relevant passages | Relevant to claim No. |
|---|---|---|
| X | CN 102145178 A (BEIJING KAWIN TECHNOLOGY SHARE-HOLDING CO., LTD.) 10 August 2011 (2011-08-10)<br>see description, pages 2-3, paragraphs 0013 and 0017-0020, page 4, paragraphs 0031-0032, embodiments 2-3, and claims 5-10 | 1-13 |
| A | CN 1651461 A (EAST CHINA UNIVERSITY OF SCIENCE AND TECHNOLOGY) 10 August 2005 (2005-08-10)<br>see claims 1-10 | 1-13 |
| A | CN 101104077 A (BEIJING ZICHEN MEDICAL BIO-TECHNOLOGY INSTITUTE) 16 January 2008 (2008-01-16)<br>see claims 1-5 | 1-13 |
| A | CN 103193879 A (SHENZHEN YATAIXING INDUSTRY LTD.) 10 July 2013 (2013-07-10)<br>see claims 1-10 | 1-13 |
| A | US 2018214561 A1 (XIAMEN SINOPEG BIOTECH CO., LTD.) 02 August 2018 (2018-08-02)<br>see claims 1-120 | 1-13 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 December 2022** | **05 January 2023** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/123400**

**C. DOCUMENTS CONSIDERED TO BE RELEVANT**

| Category* | Citation of document, with indication, where appropriate, of the relevant passages | Relevant to claim No. |
|---|---|---|
| A | CN 103804483 A (FOURTH MILITARY MEDICAL UNIVERSITY OF CHINESE PEOPLE'S LIBERATION ARMY) 21 May 2014 (2014-05-21) see claims 1-10 | 1-13 |

Form PCT/ISA/210 (second sheet) (January 2015)

**INTERNATIONAL SEARCH REPORT**
**Information on patent family members**

International application No.
**PCT/CN2022/123400**

| Patent document cited in search report | Publication date (day/month/year) | Patent family member(s) | Publication date (day/month/year) |
|---|---|---|---|
| CN 102145178 A | 10 August 2011 | None | |
| CN 1651461 A | 10 August 2005 | None | |
| CN 101104077 A | 16 January 2008 | None | |
| CN 103193879 A | 10 July 2013 | None | |
| US 2018214561 A1 | 02 August 2018 | US 2019365910 A1 | 05 December 2019 |
| | | EP 3315531 A1 | 02 May 2018 |
| | | CN 104877127 A | 02 September 2015 |
| | | WO 2016206540 A1 | 29 December 2016 |
| CN 103804483 A | 21 May 2014 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 1186121 A
- CN 109438568 A